(19) Europäisches Patentamt
European Patent Office
Office européen des brevets

(11) **EP 3 010 461 B1**

(12) **FASCICULE DE BREVET EUROPEEN**

(45) Date de publication et mention de la délivrance du brevet:
**12.09.2018 Bulletin 2018/37**

(51) Int Cl.:
*A61F 13/00* *(2006.01)*        *A61F 13/08* *(2006.01)*

(21) Numéro de dépôt: **14730901.7**

(22) Date de dépôt: **17.06.2014**

(86) Numéro de dépôt international:
**PCT/EP2014/062644**

(87) Numéro de publication internationale:
**WO 2014/202565 (24.12.2014 Gazette 2014/52)**

(54) **PROCÉDÉ POUR DÉFINIR UNE ENVELOPPE ÉLASTIQUE DE COMPRESSION-CONTENTION MODIFIANT LE COMPORTEMENT DES TISSUS BIOLOGIQUES VIVANTS ET ENVELOPPE OBTENUE**

VERFAHREN ZUR DEFINITION EINER ELASTISCHEN KOMPRESSION/STÜTZHÜLLE ZUR MODIFIZIERUNG DES VERHALTENS VON BIOLOGISCHEM LEBENDGEWEBE UND SO GEWONNENE HÜLLE

METHOD FOR DEFINING AN ELASTIC COMPRESSION/SUPPORT ENVELOPE MODIFYING THE BEHAVIOUR OF THE LIVING BIOLOGICAL TISSUES, AND ENVELOPE OBTAINED

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.06.2013 FR 1355698**

(43) Date de publication de la demande:
**27.04.2016 Bulletin 2016/17**

(73) Titulaire: **T.B.V. ET COMPRESSIONS**
**42000 Saint-Etienne (FR)**

(72) Inventeurs:
• **COUZAN, Serge**
**42000 Saint Etienne (FR)**

• **POUGET, Jean-François**
**42580 L'Etrat (FR)**

(74) Mandataire: **Cabinet Laurent & Charras**
**3 place de l'Hotel de Ville**
**CS 70203**
**42005 Saint-Etienne Cedex 1 (FR)**

(56) Documents cités:
**EP-A2- 1 240 880      WO-A1-2005/106087**
**DE-A1- 3 028 381      FR-A1- 2 633 512**
**FR-A1- 2 781 816      FR-A1- 2 801 495**

**Description**

L'invention concerne la contention-compression exercée sur une partie du corps d'un être vivant au moyen d'une enveloppe élastique réalisée par tricotage avec des fils de trame élastiques procurant une tension circonférentielle et longitudinale sur le corps vivant contraint.

Elle concerne plus particulièrement un procédé pour définir et fabriquer une enveloppe élastique de compression-contention modifiant le comportement des tissus biologiques vivants (TBV) sous-jacents d'une partie d'un corps vivant et en particulier d'un corps humain.

Actuellement, les moyens assurant une contention-compression comprennent :

- des moyens inélastiques, essentiellement constitués par des bandes qui, présentant une résistance à l'étirement , sont enroulées par spires superposées avec serrage sur une partie du corps humain. Leurs inconvénients connus sont le risque de striction localisée par leurs bords tendus, le mauvais contrôle des forces de pressions dépendant de la mise en place des spires et de la force de l'opérateur et la sensation d'inconfort qui peut en résulter.
- des moyens élastiques, essentiellement constitués par des tissus ou tricot pouvant s'allonger sous l'action d'une force de traction et qui exercent, sur la partie du corps, une action diffuse plus supportable, que le textile soit plus ou moins élastique ou plus ou moins rigide.

Normalement, on définit une enveloppe de contention-compression par les caractéristiques physiques de son matériau constitutif à savoir :

- l'élasticité qui se caractérise par l'allongement d'un matériau et se définit par la capacité du matériau à reprendre sa forme initiale après suppression d'une sollicitation ;
- l'inélasticité qui se caractérise par le non-allongement d'un matériau qui manque ou qui ne possède pas d'élasticité et correspond à la résistance du matériau ;
- l'inextensibilité se définit par ce qui ne peut être allongé par extension ou qui ne peut augmenter de volume.

Ces caractéristiques physiques sont appréciées par des appareils spécifiques dont les plus courants sont les dynamomètres. Ces mesures sont réalisées « IN VITRO » et ne peuvent pas correspondre à des mesures effectuées « IN VIVO » au sein des Tissus Biologiques Vivants.

L'étude de l'état de la technique montre qu'il existe actuellement une grande confusion dans les effets de la contention-compression et dans les caractéristiques physiques des moyens compressifs. Il en est ainsi pour les termes de résistance, de rigidité, d'élasticité et d'inélasticité appliqué par le textile compressif, qui est un concept physique, agissant sur un corps compressé, qui est un concept biologique.

Ainsi, pour caractériser une enveloppe de contention-compression il est usuel de définir sa résistance ou sa rigidité, donc de définir un paramètre mesuré hors du corps humain ou de définir une tension procurant théoriquement sur le corps humain des efforts de serrage de valeurs définies par référence à un corps tronconique régulier en appliquant la loi de Laplace. En procédant ainsi, on suppose que le corps compressé a une forme tronconique régulière et réagit de manière uniforme et homogène.

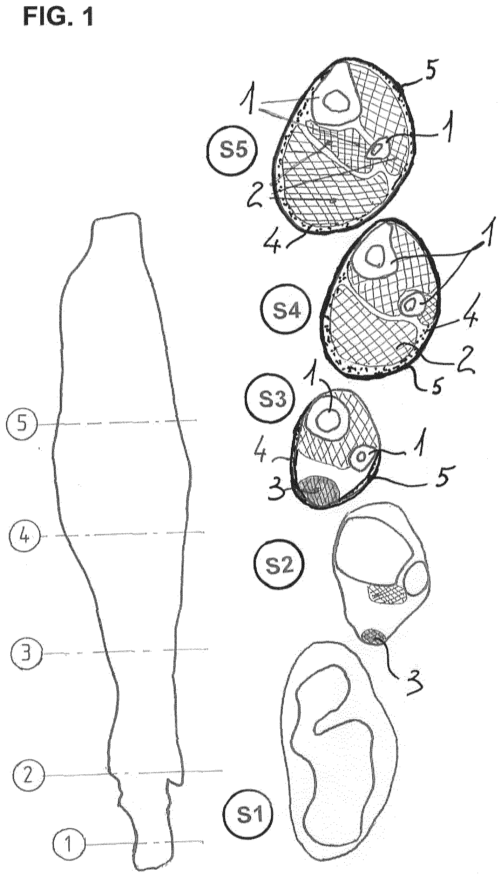

Or, c'est loin d'être le cas en raison des variations en volume, forme de la section transversale et densité des TBV du corps, comme le montrent par exemple les sections transversales S1 à S5 issues de la figure 1 représentant le profil d'une jambe vue par l'arrière. Dans ces figures et dans la description qui suit, la référence numérique 1 désigne les os, 2 les muscles, 3 le tendon d'achille, 4 la graisse et 5 la peau.

Avant de poursuivre l'énoncé des travaux exploratoires réalisés à ce jour, il est nécessaire de définir quelques unes des expressions qui vont être utilisées :

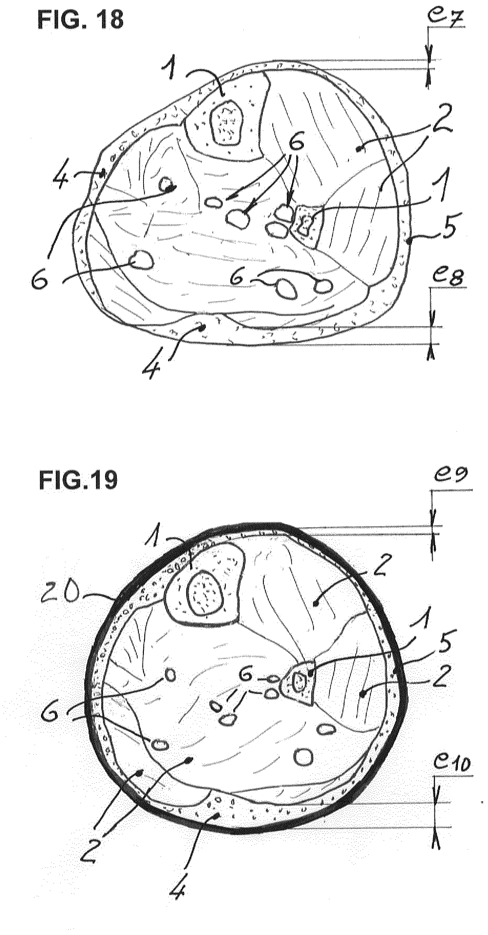

- « Corps compressif » définit le moyen textile contraignant le corps humain et dont il faut connaitre les caractéristiques pour obtenir les effets attendus.
- « Corps compressé » définit la partie du corps sur laquelle est appliquée le corps compressif et dont jusqu'à ce jour on a ignoré les nombreux effets dus à cette compression.
- « Tissus biologiques vivants », (TBV en abréviation), comprennent principalement la peau, les tissus graisseux, les muscles, aponévroses et fascias, les tendons, les nerfs, les vaisseaux et micro-vaisseaux, (artères, veines lymphatiques), les os et les tissus péri et intra-osseux. Certains de ces tissus biologiques vivants, et particulièrement la graisse sous cutanée et les muscles, absorbent à travers la peau la compression exercée sur eux par une enveloppe textile 7 et se déforment, comme montré aux figures 18 et 19, dans les sections transversales mais aussi dans toutes les directions, « à la façon d'une gélatine ». Les différents mouvements du corps compressé modifient sa forme et en particulier le rayon de courbure externe de la partie du corps, donc, par application de la loi de LAPLACE, influencent les efforts radiaux transmis à travers ces tissus biologiques vivants.

- « Masse absorbante des pressions et des déformations » est constituée par les TBV compressés et déformés sous l'effet de la compression. Cette masse est déformée en profondeur ou épaisseur, mais aussi en hauteur, largeur et de manière circonférentielle sur un segment ou sur une surface déterminée.

Cette « masse absorbante des pressions et des déformations » peut être :

- plus rigide et peu déformable, quand elle est formée par des TBV dits "durs" comme les os, les tissus péri et intra-osseux, des tendons et des tissus fibrosés,
- plus élastique et plus déformable, quand elle est formée par des TBV dits "mous" comme la peau, le tissu graisseux sous cutané, les muscles, aponévroses et fascias, les nerfs, vaisseaux et micro-vaisseaux. Ces TBV représentent ce qui peut être appelée la « masse molle ».

Actuellement, on utilise comme paramètre d'identification d'une enveloppe de contention-compression le coefficient de résistance ou de rigidité du matériau qui est déterminé par mesure à la traction par des moyens extérieurs au corps humain.

Ce « coefficient de rigidité » ne doit pas être confondu avec « l'indice de rigidité », aussi appelé « stiffness index », qui est défini par l'amplitude de la variation de pression par centimètre de changement de circonférence du corps vivant. Cet indice est mesuré « IN VIVO » sur un sujet et selon un protocole d'évaluation ne concernant qu'un site très localisé, à savoir, la surface extérieure au dessus de la peau à la partie inférieure d'une jambe humaine, au point de référence B1. Il s'agit du point de jonction entre le tendon d'Achille et les muscle du mollet exposé par la Haute Autorité de Santé dans sa définition révisée en 2010 des points de mesurage sur la jambe conformément au projet de Norme EXP ENV 12718 de 2001.

Ainsi jusqu'à présent, les différents protocoles mesurent des variations de pressions d'interface, en l'occurrence mesures de pression au point B1 entre la peau et le matériel compressif par une sonde reliée à un appareil de mesure. Ces pressions correspondent à la différence entre la pression de travail (PdT), lors d'une contraction musculaire, et la pression de repos (PdR). Selon la pratique actuelle, l'enveloppe est considérée comme « non-rigide » si PdT-PdR est inférieur à 10 et comme « rigide » si PdT-PdR est supérieur à 10.

Cette méthodologie de détermination des réactions du corps humain effectue un amalgame entre le concept physique du textile et le concept biologique de la réaction de la peau en un point précis et ponctuel, le point B1. Les conséquences physiologiques et thérapeutiques que l'on prétend en tirer n'ont jamais été prouvées. En effet, ces mesures de pressions d'interface ne sont finalement que des valeurs singulières dans le temps et dans l'espace, comme une photographie instantanée du phénomène à un endroit bien localisé.

L'invention à pour objet, après étude de la déformation par une enveloppe textile de contention-compression des tissus biologiques vivants, respectivement mous et durs, de déterminer les caractéristiques d'une enveloppe textile optimisant la déformation des tissus biologiques vivants (TBV), selon la morphologie et les besoins du traitement.

L'objet de l'invention se distingue de celui du document WO2005/106087 concernant un procédé pour définir et fabriquer une enveloppe élastique de type vetement compressif sur mesure en référence à des données dérivées du balayage de surface du corps par un scanner corporel, car ce procédé ne prend pas en considération la différence de comportements des tissus biologiques vivants soumis à une action compressive, pour éviter toute action délétère. En d'autres termes, ce document définit la fabrication de l'enveloppe sans prendre en considération son action sur les tissus biologiques vivants sous jacent

Dans la description qui suit, pour éviter la confusion entre le corps compressif textile et le corps compressé vivant (TBV), le textile ne sera pas défini par sa rigidité ou son élasticité mais par son **allongement long,** cas dans lequel il est élastique, ou par son **allongement court,** cas dans lequel il est moins élastique.

Il ressort de cet exposé que les propriétés de la contention-compression et des corps compressifs ne peuvent plus être résumées qu'aux seules actions de la loi de Laplace et de la loi de Hagen Poiseuille pour les vaisseaux sanguins, mais doivent prendre en considération la réaction particulière et le comportement des tissus biologiques vivants soumis à l'action de la contention-compression.

Ce sont ces données qu'ont fait ressortir les travaux scientifiques des Docteurs Serge COUZAN et Jean François POUGET dans leurs études scientifiques de la quantification et de la mesure de la masse absorbante des tissus biologiques vivants et de la déformation des TBV compressés.

Dans une autre phase, l'expertise des capacités d'absorption des tissus biologiques vivants a été poursuivie par une étude des capacités de déformation des TBV sous l'action d'une compression externe en utilisant la tomodensitométrie (TDM) 3 D et l'Imagerie par Résonnance Magnétique (IRM) et ceci sans intervention humaine.

L'exposé de l'état des connaissances antérieures, des travaux récents et de l'invention va être effectué en référence aux figures annexées, dans lesquelles :

Figure 1 est une vue représentant le profil d'une jambe vue par l'arrière, sans enveloppe de contention, tandis que S1, S2, S3, S4 et S5 sont des sections transversales selon les coupes 1, 2, 3, 4 et 5 identifiées sur figure 1 ;

Figures 2, 3 et 4 sont des copies de reproduction photographiques de mollet de jambe gauche vus par l'arrière de sujets, respectivement, maigre, normal et gras ;

Figures 5, 6 et 7 sont des vues en coupes anatomiques des mollets des figures 2, 3 et 4, dont la partie antérieure est tournée vers le haut ;

Figures 8, 9 et 10 représentent des vues obtenues par Tomodensitométrie (TDM) de sections d'une jambe droite, respectivement de sujet maigre (fig. 8), normal (fig. 9) ou gras (fig. 10) ;

Figures 11 à 14 sont des copies d'écran de l'échographie morphologique appliquée à des sujets respectivement maigre (fig. 11), normal (fig. 12), gras (fig. 13) et obèse (fig. 14), sous l'action d'une même pression de brassard.

Figures 15 à 17 sont des copies de vues par TDM d'une jambe droite, respectivement de sujet maigre (figure 15), normal (figure 16) et gras (figure 17) ;

Figures 18 et 19 sont des sections de jambe droite, respectivement sans aucune contention et pendant sa compression par le brassard de mesure

Figures 20, 21, 22 et 23 montrent des coupes TDM d'une jambe droite de sujet respectivement normal (fig. 20), maigre (fig. 21), gras (fig. 22) et obèse (fig. 23), quand la jambe est enveloppée dans une enveloppe de contention compression présentant une partie 7a, ayant un allongement long, et une partie 7b avec allongement court ;

Figures 24 et 25 en regard montrent deux armures d'un textile apte à assurer des allongements, respectivement longs et courts, grâce à des facteurs de couverture différents, respectivement de 37,5% et de 50% ;

Figure 26 est une vue partielle d'un tricotage fractionné et segmentaire comportant des ajouts fractionnés de fils ayant une rigidité supérieure à celle des fils formant la trame du tricot ;

Figures 27 et 28 donnent deux exemples de tableaux indiquant les valeurs de pressions minimales et maximales pour un mollet en position debout et pour un bras, avec différentes morphologies ;

Les figures 29 à 40 sont relatives à deux applications de l'invention, et plus précisément :

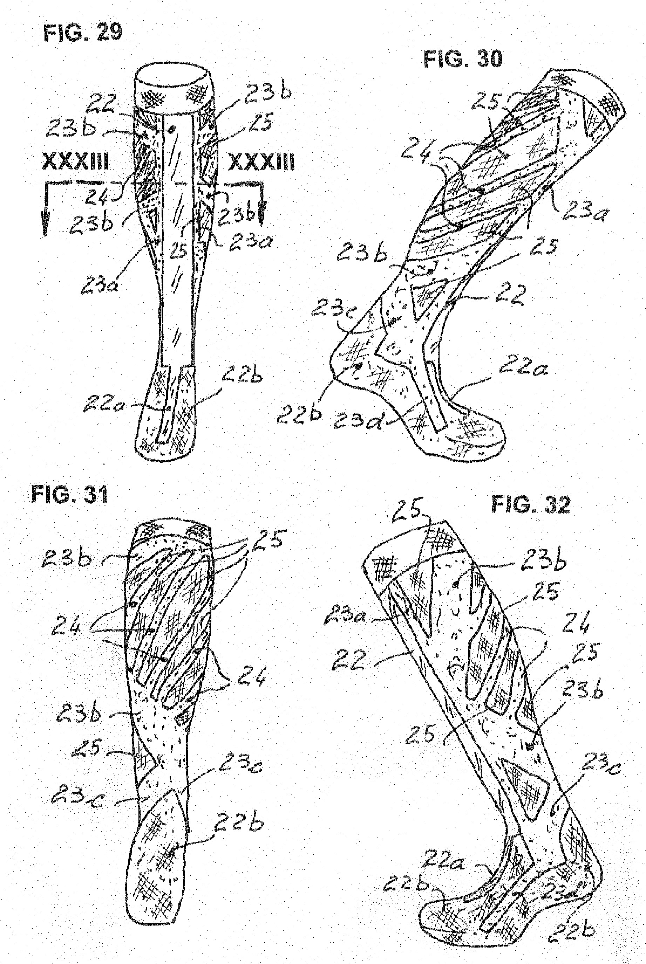

Figures 29 à 32 sont des vues en élévation, respectivement, par l'avant, par le coté intérieur, par l'arrière et par le coté extérieur, d'une chaussette gauche, pour le traitement de l'artériopathie des membres inférieurs ;

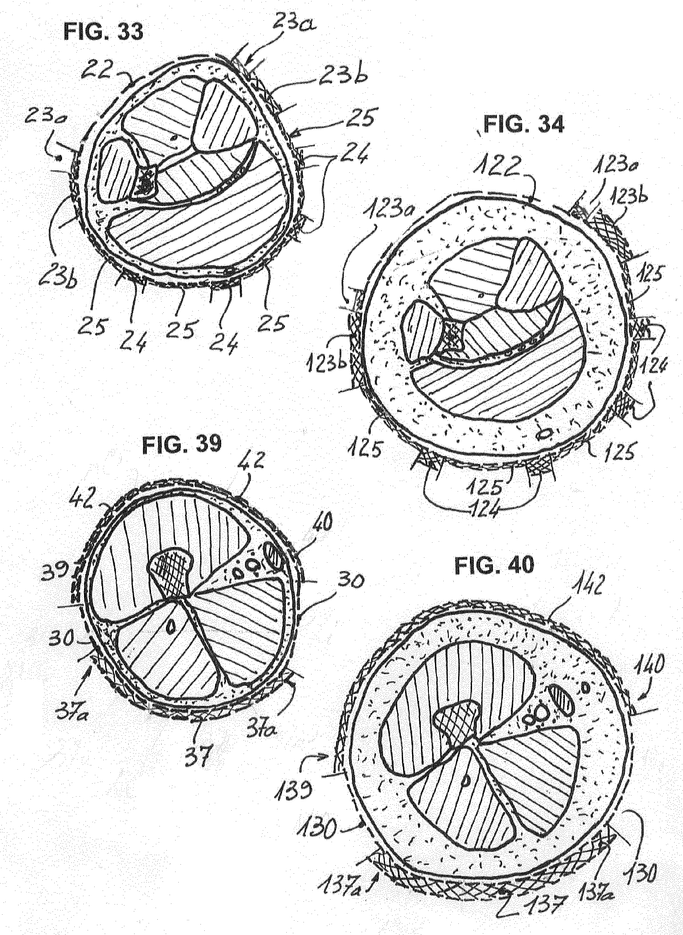

Figures 33 à 34 sont des vues en coupe suivant XXXIII-XXXIII de figure 29, respectivement, pour une jambe définie comme normale et pour une jambe définie comme grasse ;

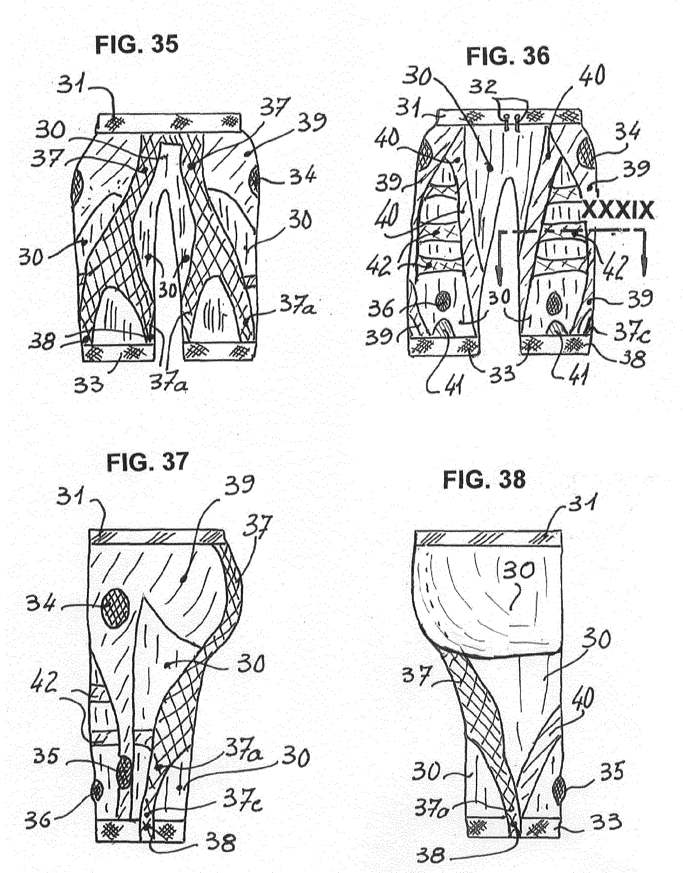

Figures 35 à 38 sont des vues en élévation d'un cuissard d'effort dynamique pour la course à pied d'un individu normal, respectivement par l'arrière, par l'avant, par le coté extérieur et par le coté intérieur, avec coupe partielle,

Figures 39 à 40 sont des vues en coupe selon XXXIX de figure 36, respectivement d'une cuisse de personne définie comme normale et d'une cuisse de personne définie comme grasse, entourée par une jambe du cuissard.

Dans les figures des sections de jambe et des coupes par Tomodensitométrie (TDM), la coupe est représentée de manière à ce que la partie antérieure de la jambe soit tournée en direction du haut de la planche de dessin et la partie postérieure en direction du bas.

Les travaux des Docteurs Serge COUZAN et Jean François POUGET ont été confirmés par deux thèses soutenues à l'Ecole Nationale Supérieure d'Ingénieur des Mines de Saint Etienne, France, par respectivement Laura BOUTEN et Laura DUBUIS, sur l'étude par IRM 2D et TDM 3D du comportement des tissus biologiques vivants soumis à la contention-compression après modélisation, respectivement en 2D puis 3D.

Ces études sur la jambe humaine ont montré des variations du coefficient de POISSON (qui permet de caractériser la contraction de la matière perpendiculairement à la direction de l'effort appliqué) selon la transmission à travers les tissus biologiques vivants, et des variations des volumes compressés dans tous les plans (profondeur, hauteur, largeur). Les conséquences de ces variations de volume vont agir sur la loi de Laplace et ainsi sur les pressions minimales et maximales à appliquer en fonction de la répartition des tissus biologiques et particulièrement du tissu graisseux sous cutané et des muscles.

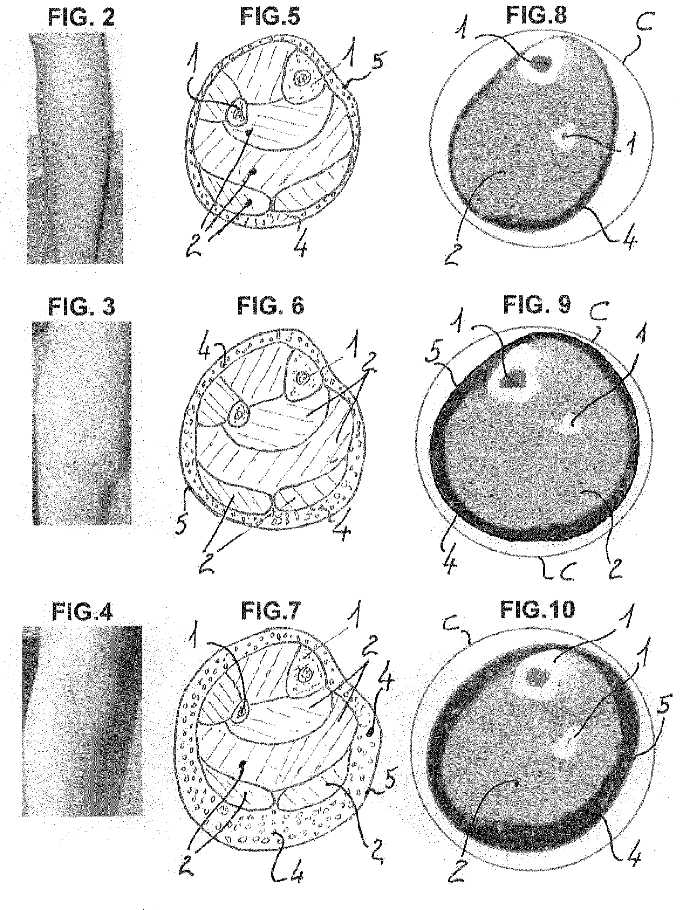

Les figures 8 à 10 annexées, qui représentent des vues par TDM de sections d'une jambe droite, respectivement de sujet maigre (fig. 8), normal (fig. 9) ou gras (fig. 10), montrent que les volumes examinés se distinguent aussi de la forme circulaire optimale C (100%) en fonction de la morphologie du sujet, montrées aux figures 2 (maigre), 3 (normal) et 4 (gras). Les figures 5 à 7, qui sont des reproductions de coupes anatomiques vues par TDM d'une jambe gauche relatives à des sujets, respectivement, maigre (fig. 5), normal (fig. 6) et gras (fig. 7), montrent plus en détail la répartition interne des tissus biologiques vivants et les variations d'épaisseur de la couche 4 de graisse, pouvant interférer dans la transmission des efforts radiaux de contention-compression.

Les Docteurs Serge COUZAN et Jean François POUGET ont continué ces travaux en procédant à une expertise des capacités d'absorption des pressions des tissus biologiques vivants sous l'action d'une compression externe. Ils ont pris en considération les rapports avec la densité, l'épaisseur, la configuration, la répartition, la localisation anatomique et la topographie (antérieure, postérieure, latérale externe ou interne) des tissus biologiques vivants sur toutes les parties du corps humain et ont concerné particulièrement la peau, la graisse sous cutanée, les muscles, les vaisseaux, les nerfs, les os et les tissus péri-osseux.

Ces études ont d'abord été réalisées à l'aide d'un échographe Doppler avec sonde HF ayant une puissance de 5 à 15 mégahertz coopérant avec un brassard avec poche gonflable et fenêtre acoustique perméable aux ondes de la sonde de mesure, tel que décrit dans le document EP1194071. Quand le brassard entourait la partie du corps concernée, par exemple bras ou jambe, il était gonflé jusqu'à ce que la pression circonférentielle (pression de déformation) écrase et déforme les tissus qui étaient examinés sur l'écran de l'échographe. Le gonflement du brassard était arrêté dès que le tissu observé, à savoir muscle, graisse, peau ou vaisseau sanguin, avait atteint le degré d'écrasement voulu. La valeur en millimètres de mercure (mm Hg) lue sur le manomètre du brassard correspondait à la pression de déformation.

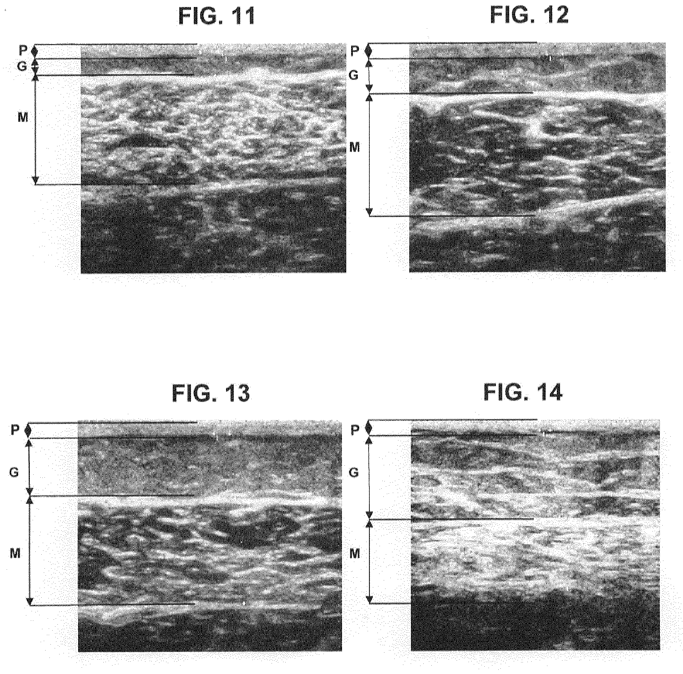

Cela est illustré par les figures 11 à 14 qui sont des copies d'écran de l'échographie morphologique appliquée à des sujets respectivement maigre (fig. 11), normal (fig. 12), gras (fig. 13) et obèse (fig. 14) sous l'action d'une même pression de brassard. Les valeurs P, G et M donnent, respectivement l'épaisseur de la peau, de la graisse et des muscles. L'examen de la croissance de l'épaisseur de la couche G entre les différentes figures indique de manière évidente que la réaction à un effort radial de compression ne peut pas être identique entre les différents sujets.

Ainsi, sous la déformation par la compression avec le brassard échographique à fenêtre acoustique, référencé 20 à la figure 19, la structure anatomique compressée va tendre à devenir plus ronde et homogène en volume en cas de TBV mous, comme le montre cette figure 19, ou moins ronde, irrégulière et hétérogène en volume en cas de TBV durs par rapport à la valeur de référence ronde et cylindrique.

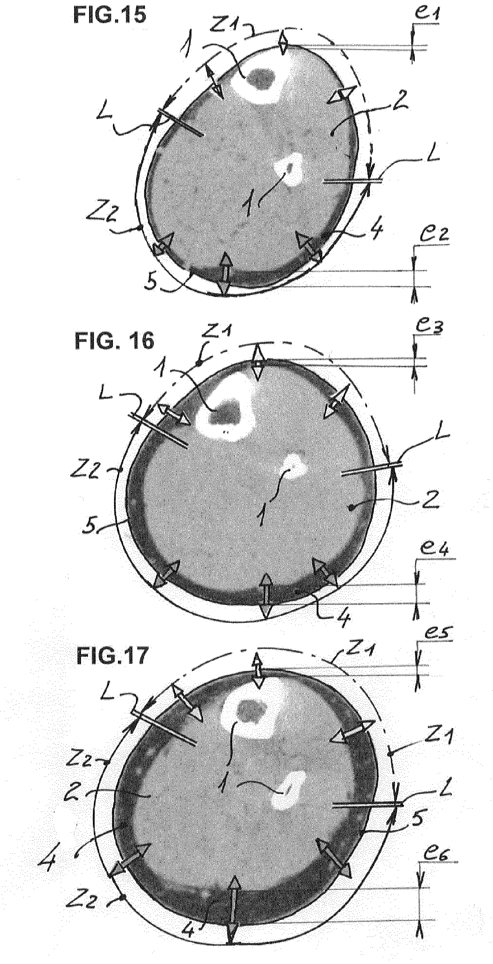

Les capacités de déformation des tissus biologiques vivants, et particulièrement de la graisse sous cutanée et des muscles (TBV dits "mous"), ont des conséquences sur la transmission des pressions du textile à travers la peau, puis en profondeur (Loi de Laplace), qui sera bonne et normale en cas de volume plus rond et homogène (avec grand rayon de courbure) et plus forte et à risque de surpression (avec petit rayon de courbure) en cas de volume moins rond, irrégulier et hétérogène. Cela est illustré aux figures 15 à 17 concernant une vue par TDM d'une jambe droite, respectivement de sujet maigre (figure 15), normal (figure 16) et gras (figure 17). Dans ces figures, les valeurs $e1$, $e3$ et $e5$, qui caractérisent l'épaisseur de la graisse dans la partie antérieure Z1 de la jambe, sont moins importantes que celles $e2$, $e4$ et $e6$ dans la partie postérieure Z2 et montrent que cette partie Z2 a des capacités d'absorption des pressions et de déformations des tissus biologiques vivants bien supérieures à celle de Z1 et dont il faut tenir compte dans la conception et la fabrication d'une enveloppe de contention-compression.

Il se révèle que les capacités d'absorption des pressions et de déformation des tissus biologiques vivants varient selon les individus mais sont corrélées à la définition classique des sujets normaux, maigres, gras, obèses dont la détermination est réalisée par des mesures anthropométriques, de l'indice de masse corporelle (IMC) et/ou de la mesure du pli cutané.

Il a donc été procédé à des mesures des pressions de compression minimales et maximales transmises à travers la peau pour agir sur les tissus biologiques vivants et les organes cibles, par exemple jambe, cuisse, bras, avant bras et bassin, en fonction des segments ou surfaces compressés. Les valeurs mesurées varient selon le segment de membre ou la surface compressée et, particulièrement pour les membres inférieurs (soumis à la pression hydrostatique), et selon les conditions de port (couché, assis, debout).

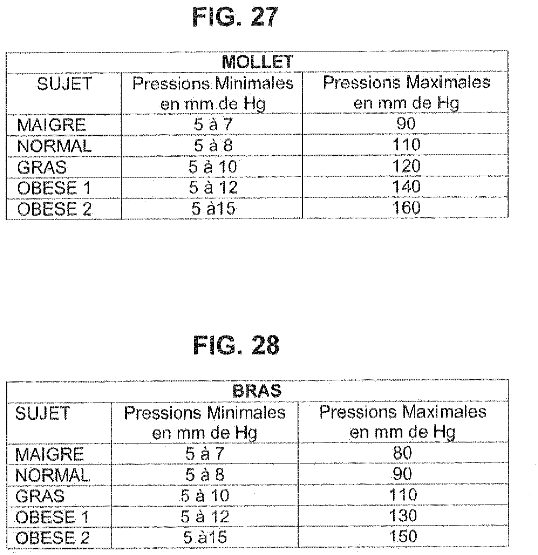

Ce processus à permis d'établir des tableaux constituant un référentiel et indiquant, pour chaque membres ou partie du corps, en rapport avec la morphologie des sujets, les valeurs des pressions minimales et maximales applicables sur les tissus biologiques TBV. Un exemple de tableaux est donné aux figures 27 et 28 annexées qui concernent, respectivement les valeurs de pressions pour un mollet en position debout et pour un bras, avec différentes morphologies.

Pour analyser de façon globale et visualiser, sans intervention humaine, le comportement de ces tissus biologiques vivants, la méthode la plus fiable a été l'étude avec l'imagerie par IRM 2D et Tomodensitométrie (TDM) 3D avec contention-compression par une enveloppe textile 7a-7b ayant une pression circonférentielle constante avec une zone textile à allongement long 7a et une autre à allongement court 7b sur le segment analysé.

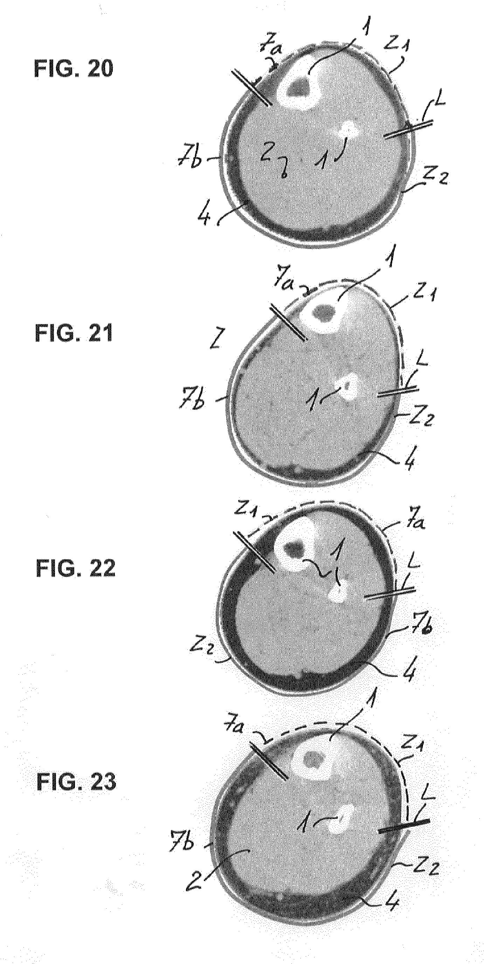

Les figures 20 à 23 montrent des coupes TDM d'une jambe droite de sujet respectivement normal (fig. 20), maigre (fig. 21), gras (fig. 22) et obèse (fig. 23). Les limites L marquent les séparations théoriques entre les zones Z1 et Z2 des tissus biologiques, respectivement durs et mous.

Dans un premier temps, le segment de corps a été entouré par une enveloppe dont le textile de contention-compression présentait une zone d'allongement court 7b et une zone d'allongement long 7a. L'enveloppe a été positionnée, avec l'allongement court 7b en regard de la zone Z1 des tissus peu malléables (TBV dits "durs"), comme les surfaces osseuses et péri-osseuses, et l'allongement long 7a en regard de la zone Z2 des tissus plus malléables (TBV dits "mous"), comme le tissu graisseux sous-cutané et les muscles.

Dans un deuxième temps, sur la même partie de corps, l'enveloppe textile a été positionnée de manière inverse, comme montré aux figures 20 à 23, c'est-à-dire avec sa partie ayant un allongement long 7a en regard des tissus peu malléables (TBV dits "durs"), comme les surfaces osseuses et péri-osseuses de la zone Z1, et la partie 7b avec l'allongement court en regard de la zone Z2 des tissus plus malléables (TBV dits "mous"), comme le tissu graisseux sous-cutané et les muscles.

L'écrasement des structures cibles (muscles, vaisseaux) et la déformation globale dans le plan circulaire ont montré des résultats différents en rapport avec le comportement des TBV (corps compressé) soumis à la compression externe par le textile ou corps compressif dont les pressions circonférentielles étaient sensiblement constantes. Les modifications du comportement des TBV étaient en rapport avec les variations segmentaires de la composition du textile à allongement court ou long en regard des TBV mous ou durs compressés.

Les valeurs des pressions de contention-compression et de déformation mesurées avec l'échographe à brassard étaient des pressions moyennes (pressions minimales et pressions maximales efficaces) selon le segment de membre analysé (mollet, cuisse, bras, avant bras, bassin ...) ont permis de déterminer les tableaux définis plus haut et d'effectuer le constat qui suit :

- Les tissus biologiques vivants de la zone Z2 et particulièrement la graisse sous-cutanée et les muscles (TBV dits "mous") absorbent les pressions exercées sur le corps par la compression du textile à travers la peau et se déforment dans toutes les directions sous l'action de cette compression externe, « comme une gélatine ». Cette déformation générale peut, pour certaine partie du corps, modifier le rayon de courbure de cette partie et avoir une incidence sur les pressions transmises à travers ces tissus biologiques vivants selon la loi de Laplace.
- A l'inverse, les tissus biologiques vivants, de la zone Z1, comme les os et les tissus péri et intra-osseux (TBV dits "durs") n'absorbent pas les pressions de la compression du textile à travers la peau et, en raison de leur consistance très dure, ne peuvent pas se déformer sous l'action de cette compression externe.

Ces constats ont permis de définir l'existence de deux types de Tissus biologiques vivants :

- Les tissus biologiques mous ayant une capacité d'absorption et donc déformables. Par leurs textures spécifiques, ils représentent une interface (de type gélatine) qui absorbe les pressions transmises par le textile compressif à travers la peau jusqu'à l'organe cible situé en profondeur. Cette absorption est dépendante de l'épaisseur et de la composition de cette interface. Ces tissus biologiques mous peuvent se déformer sous l'effet de la compression en profondeur mais également en hauteur, largeur et de manière circonférentielle sur un segment ou une surface déterminée.

Ils sont représentés principalement par la peau, le tissu graisseux en particulier sous cutané (non pathologique), les muscles, les vaisseaux et micro-vaisseaux.

- Les Tissus biologiques durs et consistants, qui sont opposés aux précédents, ne peuvent absorber que très faiblement les pressions et sont peu déformables ou malléables. Ils sont représentés principalement par les os, les tissus péri et intra-osseux, les nerfs, les aponévroses, les tendons, les tissus fibrosés, ....

Il résulte de ces observations, et il s'agit là d'une caractéristique essentielle de l'invention, que pour optimiser le comportement des tissus biologiques vivants, d'une partie d'un corps vivant, telle que mollet, cuisse, bras, avant bras, bassin, ... et, pour agir sur une zone cible plus en profondeur, sans être délétère, il faut appliquer un textile compressif **à allongement long** sur les tissus biologiques vivants (TBV) dits « **durs »** et un textile compressif à **allongement plus court** sur les tissus biologiques vivants dits « **mous** », comme le montrent les figures 20 à 23 pour la jambe,

A l'inverse, pour ne pas ou peu modifier le comportement des TBV, il faut appliquer un textile compressif à allongement court sur les TBV dits « durs » et à allongement long sur les TBV dits « mous ».

Les propriétés du textile compressif à **allongement long** et à **allongement court** sur les tissus biologiques vivants (TBV) **durs** ou **mous** ne sont pas en relation directe avec les pressions circonférentielles et longitudinales et elles peuvent en être indépendantes. Elles doivent cependant respecter les pressions minimales et maximales efficaces définies précédemment avec, pour exemple, les figures 27 et 28 annexées.

Les caractéristiques de l'enveloppe compressive doivent être adaptées aux capacités d'absorption des pressions et de déformation et à la localisation anatomique et topographique du segment ou de la surface compressée.

Ce qui est nouveau c'est la découverte des propriétés mécaniques des tissus biologiques vivants et leurs différences de comportement sous l'effet de la compression externe par un tissu élastique, ces propriétés étant modifiées lorsque le corps compressé est soumis à une contention-compression à allongement long ou à allongement plus court sur une surface donnée, et ceci même sans faire varier la pression circonférentielle exercée sur cette surface.

Ces propriétés des tissus biologiques vivants varient selon les individus mais sont corrélées à la définition classique des sujets normaux, maigres, gras, obèses en fonction des mesures anthropométriques, de l'IMC (indice de masse corporelle) et/ou de la mesure du pli cutané.

Il ressort de ces travaux que les propriétés de la contention-compression ne peuvent plus être résumées à la seule action de la loi de Laplace (ou de la loi de Hagen Poiseuille pour les vaisseaux) mais doivent prendre en compte la réaction particulière et le comportement des tissus biologiques vivants soumis à l'action de la contention-compression à allongements variables sur une surface donnée, et ceci quelque soit la pression circonférentielle exercée sur cette surface.

L'invention concerne donc un procédé pour définir une enveloppe élastique de compression-contention dénommée « corps compressif » modifiant le comportement des tissus biologiques sous jacents (TBV) d'une partie du corps humain, dénommée « corps compressé », telle que jambe, mollet, cuisse, bras, avant bras, bassin, .....

Ce procédé comprend :

- A) une phase de détermination d'un référentiel composé de tableaux donnant, pour plusieurs morphotypes humains, par exemple maigre, normal, gras et obèse, pour plusieurs parties de corps humains, par exemple jambe, mollet, cuisse, bras, avant bras, bassin, et pour plusieurs types de tissus biologiques, les valeurs minimales et maximales des pressions pouvant être appliquées sur cette partie du corps humain par une enveloppe compressive textile exerçant une pression circonférentielle et longitudinale,
- une phase de localisation topographique des zones anatomiques correspondant à ces TBV mous et durs de la partie de corps humain à compresser comme par exemple la jambe, la cuisse, le bras, l'avant bras, le genou, le coude, ..., en fonction de l'action corrective voulue ou les besoins des traitements,
- B) une phase d'adaptation de l'enveloppe aux besoins correctifs consistant :

  • D'une part, à définir les zones de l'enveloppe textile à allongement long (7a) ou à allongement court (7b) à appliquer sur les TBV mous ou durs du corps compressé en fonction de l'action corrective voulue ou les besoins des traitements, ces TBV définissant les caractéristiques de la masse absorbante molle et déformable (Z2) et dure et peu déformable (Z1) dans le corps compressé examiné,
  • d'autre part, à définir le morphotype du sujet par mesures anthropométriques, mesure de l'Indice de masse corporelle (IMC) et/ou mesure du pli cutané, ces dernières mesures définissant les caractéristiques (densité, épaisseur, répartition, configuration, localisation anatomique, topographie) de la masse absorbante molle ou dure dans le corps compressé examiné, et,
  • d'autre part, à définir la valeur des pressions efficaces comprises entre pressions minimales, maximales et de déformation à appliquer sur les masses molles et dures des TBV en fonction de l'action corrective voulue ou les besoins des traitements ;

- et C) une phase de détermination des caractéristiques de fabrication consistant, à partir i) des données relevées dans le référentiel correspondant au morphotype du sujet, ii) aux données propres au sujet et iii) à l'action corrective voulue :

  • à déterminer la tension devant être donnée aux fils de tricotage de l'enveloppe pour obtenir une tension circonférentielle et longitudinale, et,
  • pour chaque section transversale de l'enveloppe et longitudinalement sur sa longueur, à répartir, selon l'application, les zones de l'enveloppe à allongement long venant au dessus des parties résistantes et peu déformables de la partie du corps humain, et celles à allongement court venant au dessus des matières molles et plus facilement déformables pour optimiser le comportement de ces TBV.

L'invention concerne également une enveloppe élastique de compression-contention obtenue par le procédé ci-dessus, réalisée par tricotage avec des fils de trame élastiques procurant une tension circonférentielle et longitudinale et comportant, au moins dans certains segments circulaires juxtaposés, des tronçons de mailles dont l'allongement est long et des tronçons de mailles, dont l'allongement est court.

Selon l'invention, ces tronçons de mailles sont répartis, selon l'action corrective voulue ou selon les besoins d'un traitement, sur les parties de l'enveloppe destinés à venir en regard des diverses zones anatomiques compressibles et déformables (Z2), composées de peau, graisse, muscles (TBV mous), et des zones anatomiques rigides et peu déformables (Z1), composées d'os et tendons (TBV durs), de la partie de corps qu'elle doit entourer, par exemple jambe, cuisse, bras et avant bras, cette répartition des tronçons étant réalisée, selon les données du référentiel, pour contrôler la déformation des tissus biologiques contraints et le comportement physique et physiologique des TBV mous par rapport aux TBV durs sous l'effet des compressions transmises par les tronçons de mailles à allongement long et à allongement court.

Grâce à cela l'enveloppe, quelle soit en forme de chaussette, de tronçon tubulaire pour mollet ou bras, ou de cuissard, optimise la déformation des TBV en contrôlant la transmission des pressions et déformations, et en évitant tout effet délétère sur les composants des masses molles ou dures.

Il ressort de ce qui précède que, en complément des connaissances anatomiques habituelles de la partie de corps compressée, telles que la localisation de la peau, de la masse graisseuse, de la masse musculaire (TBV mous) sur lesquelles les pressions vont être facilement absorbées et celles des zones à risque de surpression et donc à respecter telles que os, tissus péri-osseux, nerfs, artères, microcirculation, lymphatiques, les travaux des docteurs COUZAN et POUGET permettent de connaitre le comportement des TBV mous et durs et les capacités de déformation de ces tissus sous l'action d'un textile compressif. Ils permettent aussi, par analyse et pour obtenir une action corrective sur certaines parties du corps, de définir les zones anatomiques sur lesquelles appliquer un textile à allongement long et les zones anatomiques sur lesquelles appliquer un textile à allongement plus court. Ceci peut être réalisé sans relation directe ou de manière indépendante des pressions circonférentielles et longitudinales du textile.

Dans une forme d'exécution, les zones à allongements courts de l'enveloppe textile sont réalisées par augmentation localisée du facteur de couverture des mailles.

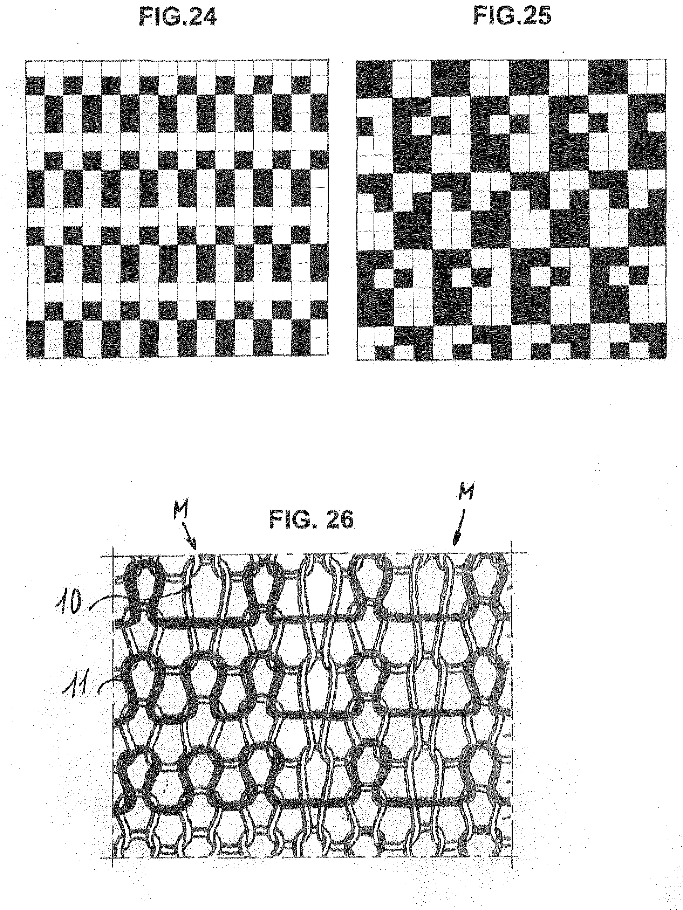

Les figures 24 et 25 montrent à titre d'exemple deux armures présentant des facteurs de couverture différents. A la figure 24, montrant l'armure d'un textile à allongement long, le facteur de couverture est de 37,5% puisque les zones vides du tricot représentent 160/256 mailles, alors que celles remplies représentent 96/256 mailles. L'armure de la figure 25, correspondant à un textile à allongement court, procure un facteur de couverture de 50%, puisque les vides comme les remplies représentent chacun 128/256, et indique que la moitié des aiguilles cueillent du fil.

En laissant plus d'air dans la même maille on obtient plus d'élasticité et un allongement plus long.

Dans une autre forme d'exécution, les zones d'allongements courts de l'enveloppe textile sont réalisées par tricotage fractionné et segmentaire avec ajout fractionné, dans certaines alimentations en fil, de fils ayant une rigidité supérieure à celle des fils formant la trame du tricot.

Cette technique assimilée à de la « broderie » est montrée à la figure 26, dans laquelle la référence 10 désigne le fil constituant les mailles M du tricot ayant un allongement long et 11 désigne le fil réalisant la broderie réduisant l'allongement de ces mailles.

Dans une autre forme d'exécution, les zones d'allongements courts de l'enveloppe textile sont obtenues par des pièces externes bloquant localement les mailles et rapportées sur chaque zone Z1 par couture ou collage.

Il est aussi possible de modifier les conditions d'allongement du textile

- En agissant sur le fil de maille, (moins d'élasticité segmentaire)
- En agissant sur le fil de trame, (moins d'élasticité circonférentielle)
- En ajoutant, dans la maille, un autre fil de trame plus rigide (moins d'élasticité circonférentielle)

L'homme de métier est en mesure de choisir les moyens de réduction d'allongement en fonction de la valeur de celui-ci, de sa localisation et de l'utilisation de l'enveloppe de contention-compression.

Il est précisé que les moyens de mesure et d'analyse des modifications d'allongement du textile sont contrôlables et quantifiables par des appareils spécifiques de type « Cetme » et des dynamomètres.

L'enveloppe selon l'invention peut faire l'objet de nombreuses applications, dont deux vont être décrites.

Les figures 29 à 32 montrent que, pour une chaussette de compression-contention, conçue pour un patient présentant une artériopathie des membres inférieurs, les zones de mailles à allongement long 7a en regard des tissus peu malléables (TBV dits "durs"), comme les surfaces osseuses et péri-osseuses et à allongement court 7b en regard des tissus plus malléables (TBV dits "mous"), comme le tissu graisseux sous-cutané et les muscles s'étendent sur l'ensemble de la chaussette pour former des bandes réparties spatialement, et de manière asymétrique entre la jambe droite et la jambe gauche.

Ainsi à ces figures, représentant une chaussette pour jambe gauche, 22 désigne une bande textile à allongement long venant sur l'avant de la jambe, au dessus de la crête tibiale, et se prolongeant en 22a au dessus du tendon extenseur et du nerf fibulaire. Une partie du pied et de la cheville est à allongement long 22b mais d'une valeur inférieure à 22a.

23a désigne deux bandes textiles à allongement court bordant la bande 22 et se prolongeant latéralement par des bandes larges 23b, formant sur la partie arrière un X encadrant le mollet et le contraignant plus fortement sur son coté extérieur que sur son coté intérieur. Chacune des deux extrémités inférieures des branches 23b, venant au-dessus de la partie inférieure du muscle soléaire et de la lame transverse, se mêle à une branche large 23c en formant un autre X. Celui-ci, visible figure 31, recouvre la jonction du calcanéum et la pointe du soléaire. Chaque branche large 23c s'étend au dessus d'une malléole, latérale ou et médiale, et diverge par une bande étroite 23d s'étendant au-dessus, respectivement, du tendon cours fibulaire ou du tendon du muscle tibial antérieur longeant le pied.

Enfin, les bandes 23b supérieures et celles inférieures sont reliées par un réseau des bandes étroites 24 qui, espacées et en spirale allant du haut du coté intérieur au bas du coté extérieur en recouvrant les muscles gastrocnémien latéral et gastrocnémien médial, ont un effet légèrement contraignant sur le mollet.

Entre ces diverse bandes la chaussette comprend un textile élastique 25 présentant des mailles à allongement intermédiaire.

Cette chaussette de compression-contention, conçue pour un patient présentant une artériopathie des membres inférieurs a pour action d'optimiser le fonctionnement musculaire et vasculaire fragilisé par la carence d'apport en oxygène en rapport avec la mauvaise vascularisation induite par la diminution du débit artériel arrivant au niveau des différents TBV mous et durs de la jambe et du pied. La traduction clinique de l'artériopathie des membres inférieurs est la claudication (le plus souvent du mollet) à la marche c'est-à-dire des douleurs musculaires obligeant le patient de s'arrêter de marcher après une certaine distance de marche. Actuellement, la contention-compression dégressive avec des pressions maximales en cheville est, selon les dernières recommandations de la Haute Autorité de Santé (HAS), contre indiquée en cas d'index de pression systolique en cheville (IPS ch.) < à 0,60 et sous surveillance médicale stricte en cas d'IPS ch. compris entre 0,60 et 0,90. Les avantages de la nouvelle chaussette à allongement variable et à pressions spécifiques (compression progressive) est d'optimiser le comportement physiologique des différents TBV compressés, d'améliorer ainsi le rendement musculaire et vasculaire de ces patients sans exercer d'effet délétère.

Les variations d'allongement du textile vont être décrites en référence à la figure 33, représentant une section transversale suivant XXXIII-XXXIII de figure 33, d'une jambe équipée de la chaussette des figures 29 à 32. On retrouve, sur cette figure, les différents éléments de la chaussette, à savoir la bande textile 22, les bandes 23a, 23b et 24, ainsi que le textile élastique 25.

La bande textile 22 possède un allongement long noté AL(N), le suffixe « N » indiquant que cette chaussette est destinée à une personne de corpulence normale. De façon analogue, on note AC1 (N) l'allongement des bandes 23a, 23b et 24, ainsi que AC2(N) l'allongement du textile 25. Par convention l'allongement intermédiaire du textile 25 est assimilé à un allongement « court », c'est-à-dire qu'on parle d'un premier et d'un second allongement court, respectivement AC1 et AC2.

Le tronçon à allongement long, destiné à venir au contact des TBV durs, est donc formé par la bande 22. De plus le tronçon à allongement court, destiné à venir au contact des TBV mous, est formé par les bandes 23a, 23b et 24, ainsi que par le textile 25.

La figure 34 représente une coupe transversale d'une chaussette analogue à celle de la figure 33, mais qui est destinée à une personne de corpulence dite « grosse ». Le plan de coupe de cette figure 34 est le même que celui de la figure 33.

On suppose par ailleurs que ces chaussettes doivent être portées par des personnes de même taille, dont seules les morphologies sont différentes. Ces deux chaussettes ont par conséquent la même dimension longitudinale, ou longueur, mais des périmètres qui augmentent avec la corpulence du sujet.

Les éléments de la figure 34, analogues à ceux de la figure 33, y sont affectés des mêmes numéros de référence augmentés de 100. Comme ci-dessus, on note AL(G) l'allongement de la bande 122, AC1(G) l'allongement des bandes 123a, 123b et 124, ainsi que AC2(G) l'allongement du textile 25. La notation « G » signifie que cette chaussette est destinée à une personne de corpulence grosse.

Selon l'invention, l'allongement court 7b est utilisé pour agir sur les différents constituants de la masse molle des TBV dit mous. Cette masse molle qui va absorber les pressions du textile transmises à travers la peau est d'autant plus importante que le sujet est gros (figures 15 à 17). A pressions circonférentielles égales, il faudra appliquer un textile avec un allongement d'autant plus court que le sujet est gros. Ainsi, la valeur absolue de chacun des deux allongements courts AC1 et AC2 diminue avec la corpulence du sujet, à savoir que :

$$AC1(N) > AC1(G) \text{ et } AC2(N) > AC2(G)$$

Par ailleurs, la valeur de l'allongement long peut également diminuer avec la corpulence du sujet, à savoir que : AL(N) >AL(G). Néanmoins on peut aussi prévoir que la valeur de l'allongement long est identique entre les deux chaussettes.

De façon plus générale, on peut prévoir un ensemble de chaussettes comprenant, outre celles des figures 33 et 34, deux autres chaussettes non représentées adaptées à des sujets respectivement maigre et obèse. Comme ci-dessus, on note pour chaque chaussette AL(M) et AL(O) les valeurs d'allongement long, AC1(M) et AC1(O) les valeurs du premier allongement court, ainsi que AC2(M) et AC2(O) les valeurs du deuxième allongement court. Les suffixes « M » et « O » sont relatifs aux corpulences respectivement maigre et obèse.

En généralisant les relations ci-dessus, l'invention prévoit que la valeur des deux allongements courts diminue avec la corpulence du sujet, à savoir que :

$$AC1(M) > AC1(N) > AC1(G) > AC1(O) \text{ (relation 1)}$$

et

$$AC2(M) > AC2(N) > AC2(G) > AC2(O) \text{ (relation 2) ;}$$

relations dans lesquelles les notations (M), (N), (G) et (O) correspondent aux morphologies dites, respectivement, maigre, normale, grasse et obèse.

En d'autres termes, ces allongements sont de plus en plus courts, au fur et à mesure que la corpulence des sujets augmente.

Comme vu ci-dessus, la valeur de l'allongement long peut être constante pour les quatre chaussettes. Elle peut diminuer d'une chaussette à l'autre, avec l'augmentation de la corpulence. On peut aussi prévoir différentes valeurs de cet allongement long, plus élevées pour les sujets maigre et normal, plus faibles pour les sujets gros et obèse.

Les relations mathématiques (1) et (2) ci-dessus sont vérifiées sur au moins une partie substantielle, par exemple pour une jambe environ 66% (2/3), de la longueur des chaussettes. Ces relations (1) et (2) peuvent ne pas être vérifiées en particulier sur les zones d'extrémité de la chaussette, assurant notamment son attache sur le sujet.

Par nature, le périmètre des chaussettes augmente avec la corpulence des sujets. Il en va de même, en coupe transversale, pour la longueur du tronçon à allongement long formé par la bande 22, ainsi que pour la longueur du tronçon à allongement court formé par les bandes 23a, 23b et 24, ainsi que le textile élastique 25.

Selon une caractéristique avantageuse de l'invention, le rapport entre la longueur du tronçon à allongement court et la longueur du tronçon à allongement long est constant, pour toutes les gammes d'enveloppes, en un point donné de ces enveloppes. Dans l'exemple décrit et représenté, ce rapport est voisin de 2, à savoir que le tronçon à allongement court s'étend sur une distance double de celle du tronçon à allongement long.

Les figures 35 à 40 sont relatives à l'application de l'invention à la fabrication d'un cuissard d'effort dynamique, par exemple pour la course à pied ou la marche rapide.

Il s'agit, selon l'exemple d'application, d'un cuissard ¾ prenant le bassin jusqu'aux crêtes iliaques, recouvrant les cuisses et s'arrêtant au dessous du genou, par exemple à 10 centimètres, ou au dessus. Destiné aux personnes en mouvement, il possède une architecture combinant, suivant les régions anatomiques, des zones de rigidité et d'élasticité variables avec au moins trois types d'allongement.

La décomposition du mouvement lors de la course montre qu'il y a d'abord une flexion de la hanche et du genou, puis une extension du genou. Pour augmenter le rendement de l'appareil musculo-tendineux en se servant du jeu musculo-squelettique, le cuissard est ancré sur le bassin, au niveau des crêtes iliaques, par exemple par une ceinture souple 31 à lacets 32, et sur les genoux par un anneau de maintien 33.

Il comprend donc un textile de base 30 ayant un allongement long, noté AL'2(N) s'étendant verticalement entre la ceinture 31, et l'anneau inférieur de maintien 33 venant sur les insertions des courts et longs biceps. Ce textile 30 comporte des zones de grands allongements, noté AL'1(N), dont celles 34 sont disposées dans les parties de chacune des jambes venant sur le grand trochanter, celles 35 sur les parties venant sur les condyles latéraux et celles 36 sur les rotules.

Il comporte sur sa partie arrière des bandes 37 à allongement court, notées AC'2, et s'étendant vers le bas depuis la partie centrale de la ceinture 31 en formant deux bras 37. Ceux ci passent sur les muscles fessiers et se divisent, sur chaque jambe, en deux branches 37a s'écartant sur les cotés de la jambe jusqu'aux zones d'insertion 38 du semi tendineux et du biceps fémoral.

Sur la partie avant de chaque jambe, le cuissard comporte, d'une part, une bande 39 allant en descendant en biais de la partie latérale extérieure de la ceinture 31 vers la parie intérieure du bandeau 33, d'autre part, une bande latérale interne 40, allant en biais de la ceinture 31 au bord latéral interne du bandeau 33, et, de plus, trois barreaux horizontaux 42 s'étendant entre les bandes 39 et 40. Les bandes 39, 40 et barreaux 42, ont un allongement court notés AC1.

Chaque jambe comporte aussi, à sa partie inférieure frontale, une zone centrale 41 venant sur l'insertion tibiale et présentant un allongement court de type AC1, et, à sa partie latérale inférieure l'ancrage 37c de l'extrémité de la branche 37a ayant un allongement court de type AC2.

La figure 39 est une coupe transversale, suivant le plan de coupe XXXIX à la figure 36, du cuissard. On retrouve, sur cette figure, les différents éléments du cuissard, à savoir les bandes respectivement bandes avant 40 et barreaux 42, bandes arrière 37 et 37a, ainsi que bandes latérales 39.

Les bandes 39, 40 et barreaux 42 possèdent un même allongement court et sont notés AC1 (N), la notation « N » étant ajoutée pour rappeler que ce cuissard est destiné à une personne de corpulence normale. Cet allongement court AC1'(N) est par exemple légèrement inférieur à celui long AL(N) de la bande 22 décrite dans la chaussette de la précédente application.

En d'autres termes, ces bandes 39, 40 et 42 sont un peu moins rigides que la bande 22.

De façon analogue, on note AC'2(N) l'allongement de la bande arrière 37-37a ainsi que AC'1(N) l'allongement des bandes en avant 39, 40 et 42. Comme dans le cas de la chaussette, l'allongement intermédiaire des bandes avant 39, 40 et 42 est assimilé à un allongement « court ». Les allongements AC'1(N) et AC'2(N) sont par exemple identiques respectivement à AC1(N) et AC2(N) décrits ci-dessus. En d'autres termes les bandes 39 et 40 sont aussi rigides que les bandes 23, 23a et 24, alors que la bande 30 est aussi rigide que le textile 25. Le tronçon à allongement intermédiaires est donc formé par les bandes 39, 40 et 42, tandis que le tronçon à allongement court est formé par les bandes 37.

Quand le cuissard est passé sur une personne en mouvement à sa taille, il améliore le rendement des ischio-jambiers et du quadriceps disposés dans la loge antérieure et postérieure de la cuisse. Lors de la flexion de la cuisse, la zone d'allongement court du cuissard située en arrière des ischio-jambiers comprime ces muscles, améliore le retour veineux et diminue les vibrations et oscillations dans cette zone, tandis que la zone antérieure qui bénéficie de deux zones de rigidité intermédiaire mais aussi d'élasticité, est maintenue sans que le sportif ne soit gêné. Lors de l'extension, il y a transfert des pressions du textile élastique de l'avant vers l'arrière en facilitant la contraction du quadriceps et en permettant un meilleur maintien des ischio-jambiers.

Latéralement, l'action se situe essentiellement au niveau des muscles fessiers et du tenseur du « fascia lata » avec un rôle de stabilisation assuré par les larges bandes 39 et 40 ayant un allongement intermédiaire. Les zones à allongement long donc élastiques aménagées en regard des reliefs osseux du grand trochanter et du condyle fémoral évitent l'irritation des tendons du moyen fessier et du tenseur du « fascia lata ».

Les points d'ancrage, constitués par les crêtes iliaques et les genoux, permettent une adaptation de complexe latéral aux mouvements de flexion-extension , mais aussi de rotation en suivant au plus près les mouvements des tendons et des muscles latéraux, en les maintenant sans els contraindre.

Au niveau du genou, le cuissard se comporte comme une orthèse dynamique souple avec rôle de maintien tendineux et ligamentaire mais aussi comme point d'ancrage amplifiant les effets du tricotage spécifique sur al cuisse.

Le creux poplité présente une zone élastique protégeant les vaisseaux et nerfs particulièrement exposés.

La zone médiale de la cuisse, la partie antérieure du pelvis, le périnée et la zone du pli fessier bénéficient d'une zone d'élasticité intermédiaire assurant un maintien dynamique non contraignant.

Un tel cuissard apporte les avantages suivants :

- Amélioration du rendement musculaire avec diminution des risques de crampes et de blessures ;
- Protection tendineuse, avec diminution du risque de syndrome de l'essuie-glace, de tendinite rotulienne ou du moyen fessier ;
- Renforcement articulaire ;
- Amélioration de la circulation artérielle et veineuse.

Il ressort que par l'architecture du cuissard lui permet de modifier le comportement des TBV sous jacents à différentes fins, y compris dans le domaine du sport.

Selon l'invention le cuissard décrit jusqu'alors pour une personne normale peut bien entendu être adapté pour une personne maigre, grosse ou obèse. Ainsi, la figure 40 est similaire à la figure 39 mais correspond à un cuissard pour personne de corpulence dite « grosse ». Le plan de coupe est le même qu'à la figure 39. On suppose par ailleurs que ces cuissards doivent être portés par des personnes de même taille, dont seules les morphologies sont différentes. Ces deux cuissards ont par conséquent la même longueur, mais des périmètres qui augmentent avec la corpulence du sujet.

Les éléments de la figure 40, analogues à ceux de la figure 39, portent les mêmes numéros de référence augmentés de 100. Comme ci-dessus, on note AL'2(G) l'allongement long des bandes 130, et AC'1(G) l'allongement court des bandes 139, 140 et 142, ainsi que AC'2(G) l'allongement de la bande 137. La notation « G » signifie que ce cuissard est destiné à une personne de corpulence grosse.

Comme dans le cas de la chaussette des figures précédentes, la valeur de chacun des deux allongements courts est plus basse pour le cuissard destiné à la personne de corpulence grosse. Par ailleurs, la valeur de l'allongement long peut également diminuer avec la corpulence du sujet. Cet allongement long peut aussi être identique entre les deux cuissards.

De façon générale, comme pour les chaussettes ci-dessus, on peut prévoir un ensemble de cuissards conforme à l'invention. Cet ensemble comprend, outre ceux (N) et (G) des figures 39 et 40, deux autres cuissards non représentés adaptés à des sujets respectivement maigre et obèse. Comme ci-dessus, on note pour chaque cuissard AL'(M) et AL'(O) les valeurs d'allongement long, AC'1(M) et AC'1(O) les valeurs du premier allongement court, ainsi que AC'2(M) et AC'2(O) les valeurs du deuxième allongement court.

De façon analogue à ce qui a été décrit pour les chaussettes, la valeur des deux allongements courts diminue avec la corpulence du sujet, à savoir et avec les mêmes notations que :

$$AC'1(M) > AC'1(N) > AC'1(G) > AC'1(O) \text{ (relation 1')}$$

et

$$AC'2(M) > AC'2(N) > AC'2(G) > AC'2(O) \text{ (relation 2').}$$

Comme vu ci-dessus, la valeur de l'allongement long peut être constante pour les quatre cuissards. Elle peut diminuer d'un cuissard à l'autre, avec l'augmentation de la corpulence. On peut aussi prévoir différentes valeurs de cet allongement long, plus élevées pour les sujets maigre et normal, plus faibles pour les sujets gros et obèse.

De façon plus générale, l'invention prévoit un ensemble d'enveloppes, qui peuvent recouvrir les mollets ou les cuisses, comme les chaussettes ou les cuissards ci-dessus, mais aussi d'autres parties du corps.

De façon typique, l'enveloppe de référence est celle destinée à une personne de corpulence normale. Le tronçon à allongement long, destiné à venir au contact des TBV durs, peut comprendre une unique valeur d'allongement, ou bien deux valeurs voisines l'un de l'autre. Le tronçon à allongement court, destiné à venir au contact des différents constituants des TBV mous, plus nombreux et plus volumineux, peut en revanche comprendre plus de deux valeurs d'allongement, par exemple jusqu'à quatre. De plus, ces valeurs d'allongement court peuvent être sensiblement différentes les unes des autres.

L'ensemble d'enveloppes selon l'invention est alors développé en réalisant typiquement trois autres enveloppes de périmètre différent, dont la ou chaque valeur d'allongement court varie en fonction de la corpulence, selon les relations (1), (1'), (2) et (2') ci-dessus. Comme expliqué également ci-dessus, la ou chaque valeur d'allongement long peut varier en fonction de la corpulence, ou bien être constante d'une enveloppe à l'autre.

Dans cet esprit l'invention a donc aussi pour objet un ensemble d'enveloppes de contention-compression avec contrôle de la déformation des tissus biologiques contraints, chaque enveloppe étant réalisée par tricotage avec des fils de trame élastiques procurant une tension circonférentielle et longitudinale.

Dans cet ensemble composé de plusieurs enveloppes présentant des périmètres différents en fonction des morphotypes des sujets, chaque enveloppe présente en section transversale un premier tronçon de mailles dont l'allongement est long, ce premier tronçon étant destiné à venir au contact des TBV durs, et au moins un second tronçon de mailles dont l'allongement est court, ce second tronçon étant destiné à venir au contact des TBV mous, tandis que, sur au moins une partie de la dimension longitudinale des enveloppes, l'allongement de chaque tronçon court diminue d'une enveloppe à l'autre, lorsque le périmètre de l'enveloppe augmente selon les relations suivantes :

- relation 1 : AC1 (M) > AC1 (N) > AC1 (G) > AC1 (O),
- relation 2 : AC2(M) > AC2(N) > AC2(G) > AC2(O),
- relation 1' : AC'1 (M) > AC'1 (N) > AC'1 (G) > AC'1 (O), et
- relation 2' : AC'2(M) > AC'2(N) > AC'2(G) > AC'2(O).

Il ressort de ce qui précède, que l'invention fait intervenir un premier type de gradient d'allongement, entre différentes enveloppes destinées à des sujets de corpulence variable. L'invention peut aussi être fondée sur la mise en pratique d'un autre type de gradient, dans une même enveloppe.

En effet, on a jusqu'à présent fait référence à un morphotype unique pour un sujet donné. Cependant, dans certaines circonstances, un sujet peut présenter localement un morphotype spécifique, qui est différent du morphotype principal du reste de son corps. Ainsi, dans le cas d'un traumatisme, un sujet maigre ou normal possède un morphotype local obèse, dans la zone de ce traumatisme. On citera, par exemple, le cas d'un hématome ou d'un oedème survenant suite à une entorse, un traumatisme ou encore de séquelles post-opératoires.

La chaussette des figures 29 à 34 peut être réalisée de façon adaptée, en fonction de la localisation d'un tel traumatisme. On suppose ici qu'un sujet de morphotype normal a subi une entorse de la cheville. La plus grande partie de cette chaussette possède alors les valeurs d'allongements courts ci-dessus, à savoir AC1(N) et AC2(N). En revanche, au niveau de la cheville, cette chaussette présente un allongement court qui, compte tenu du gonflement et de l'augmentation de l'épaisseur de la masse absorbante des pressions en rapport avec l'hématome ou l'oedème, peut être assimilé, par exemple, à AC1(O) et/ou AC2(O) théoriquement adapté à un sujet obèse.

On peut également réaliser le cuissard des figures 35 à 40, selon la localisation d'un tel traumatisme. On suppose ici qu'un sujet de morphotype normal a subi une intervention sur varices avec ablation ou occlusion de la grande saphène. La plus grande partie de ce cuissard possède alors les valeurs d'allongements courts décrites, à savoir AC'1(N) et AC'2(N). En revanche, au niveau du trajet de la grande saphène occluse ou enlevée qui va être le siège d'un hématome ou d'un oedème augmentant l'épaisseur de la masse absorbante des pressions, ce cuissard présente un allongement court qui peut être assimilé, par exemple à AC'1 (O) et/ou AC'2(O) théoriquement adapté à un sujet obèse.

Il en est de même en cas d'intervention sur varices avec ablation ou occlusion de la petite saphène correspondant à la jambe. La plus grande partie de la chaussette possède alors les valeurs d'allongements courts décrites, à savoir AC1(N) et AC2(N). En revanche, au niveau du trajet de la petite saphène occluse ou enlevée qui va être le siège d'un hématome ou d'un oedème augmentant l'épaisseur de la masse absorbante des pressions, la chaussette présente un allongement court qui peut être assimilé, par exemple à AC1(O) et/ou AC2(O) théoriquement adapté à un sujet obèse. Cet allongement court peut résulter d'une bande rigide cousue ou collée localement sur la chaussette.

**Revendications**

1. Procédé pour définir une enveloppe élastique de compression-contention modifiant le comportement des tissus biologiques vivants (TBV) sous jacents d'une partie du corps humain, **caractérisé en ce qu'**il comprend :

    A) une phase :

        • de détermination d'un référentiel composé de tableaux donnant, pour plusieurs morphotypes humains, par exemple maigre (M), normal (N), gras (G) et obèse (O), pour plusieurs parties de corps humains, par exemple jambe, mollet, cuisse, bras, avant bras, bassin, et pour plusieurs types de tissus biologiques, mous ou durs, les valeurs minimales et maximales des pressions pouvant être appliquées sur cette partie du corps humain par une enveloppe compressive textile (7) exerçant une pression circonférentielle et longitudinale, et,
        • de localisation topographique des zones anatomiques correspondant à ces TBV mous et durs de la partie de corps humain à compresser comme par exemple la jambe, la cuisse, le bras, l'avant bras, le genou, le coude, ..., en fonction de l'action corrective voulue ou les besoins des traitements,

    B) une phase d'adaptation de l'enveloppe aux besoins correctifs consistant :

        • D'une part, à définir les zones de l'enveloppe textile à allongement long (7a) ou à allongement court (7b) à appliquer sur les TBV mous ou durs du corps compressé en fonction de l'action corrective voulue ou les besoins des traitements, ces TBV définissant les caractéristiques de la masse absorbante molle et déformable (Z2) et dure et peu déformable (Z1) dans le corps compressé examiné,
        • d'autre part, à définir le morphotype du sujet par mesures anthropométriques, mesure de l'Indice de masse corporelle (IMC) et/ou mesure du pli cutané, ces dernières mesures définissant les caractéristiques (densité, épaisseur, répartition, configuration, localisation anatomique, topographie) de la masse absorbante molle ou dure dans le corps compressé examiné, et,
        • de plus, à définir la valeur des pressions efficaces comprises entre pressions minimales, maximales et de déformation à appliquer sur les masses molles et dures des TBV en fonction de l'action corrective voulue ou les besoins des traitements ;

    C) une phase de détermination des caractéristiques de fabrication de l'enveloppe (7) consistant, à partir i) des données relevées dans le référentiel correspondant au morphotype du sujet, ii) aux données propres au sujet et iii) à l'action corrective voulue :

        • à déterminer la tension devant être donnée aux fils de tricotage pour obtenir dans l'enveloppe (7) la tension circonférentielle et longitudinale voulue, et,
        • pour chaque section transversale de l'enveloppe (7) et sur sa longueur, à répartir, selon l'application, les zones de l'enveloppe à allongement long (7a) venant au dessus des parties résistantes et peu déformables (Z1) de la partie du corps humain (TBV durs), et celles à allongement court (7b) venant au dessus des zones (Z2) des matières molles et plus facilement déformables des TBV mous, ceci pour optimiser le comportement des TBV par contrôle de la transmission des pressions et des déformations dans les TBV.

2. Procédé pour définir une enveloppe élastique de compression-contention modifiant le comportement des tissus biologiques sous jacents (TBV) d'une partie du corps humain, selon la revendication 1 **caractérisée en ce qu'**il consiste à choisir entre quatre morphotypes respectivement, maigre, normal, gras et obèse.

3. Procédé pour définir une enveloppe élastique de compression-contention modifiant le comportement des tissus biologiques sous jacents (TBV) d'une partie du corps humain, selon la revendication 1 **caractérisée en ce que** la répartition dans l'enveloppe (7) des zones, respectivement à allongement long (7a) et des zones à allongement court (7b), est obtenue par modifications du facteur de couverture des mailles du tricot constituant l'enveloppe (7).

4. Procédé pour définir une enveloppe élastique de compression-contention modifiant le comportement des tissus biologiques sous jacents (TBV) d'une partie du corps humain, selon la revendication 1 **caractérisée en ce que** la répartition dans l'enveloppe (7) des zones, respectivement à allongement long (7a) et des zones à allongement court (7b), est obtenue par modifications des caractéristiques du fil de trame.

5. Procédé pour définir une enveloppe élastique de compression-contention modifiant le comportement des tissus biologiques sous jacents (TBV) d'une partie du corps humain, selon la revendication 1 **caractérisée en ce que** la répartition dans l'enveloppe (7) des zones, respectivement à allongement long (7a) et des zones à allongement court (7b), est obtenue par ajout, par la technique de broderie, de mailles supplémentaires sur un segment de surface.

6. Procédé pour définir une enveloppe élastique de compression-contention modifiant le comportement des tissus biologiques sous jacents (TBV) d'une partie du corps humain, selon la revendication 1 **caractérisée en ce qu'**on définit un morphotype principal du sujet, ainsi qu'un morphotype local de ce sujet, différent du morphotype principal, au niveau d'une zone traumatique, et **en ce qu'**on donne à l'enveloppe des valeurs d'allongement spécifiques, dans la région de cette enveloppe destinée à recouvrir la zone traumatique du sujet.

7. Enveloppe de compression obtenue par le procédé selon la revendication 1 et l'une quelconque des revendications 2 à 5, cette enveloppe étant réalisée par tricotage avec des fils de trame élastiques procurant une tension circonférentielle et longitudinale et comportant, au moins dans certains segments circulaires juxtaposés, des tronçons de mailles (7a, 7b) dont l'allongement est long et des tronçons de mailles (7a), dont l'allongement est court, ces tronçons (7a, 7b) étant répartis, selon l'action corrective voulue ou selon les besoins d'un traitement, sur les parties de l'enveloppe destinés à venir en regard des diverses zones anatomiques compressibles et déformables (Z2), composées de peau, graisse, muscles (TBV mous), et des zones anatomiques rigides et peu déformables (Z1), composées d'os et tendons (TBV durs), de la partie de corps qu'elle doit entourer, par exemple jambe, cuisse, bras et avant bras, cette répartition des tronçons (7a et 7b) étant réalisée, selon les données du référentiel, pour contrôler la déformation des tissus biologiques contraints et le comportement physique et physiologique des TBV mous par rapport aux TBV durs sous l'effet des compressions transmises par les tronçons de mailles (7a et 7b) à allongement longs et à allongement court, **caractérisée et en ce que** dans son application à une chaussette pour la prise en charge de l'artériopathie des membres inférieurs l'enveloppe comprend :

   - une bande textile à allongement long (22) destiné à venir contre l'avant de la jambe et les TBV durs,
   - deux bandes (23a) à allongement court bordant celle (22) à allongement long et se prolongeant par des bandes larges (23b) formant, d'une part et sur la partie arrière, un X supérieur encadrant le mollet et le contraignant plus fortement sur son coté extérieur, et d'autre part, sur sa partie inférieure et avec des branches (23c), un autre X recouvrant la jonction du calcanéum et la pointe du soléaire,
   - dans l'intervalle entre les branches (23b) formant un X supérieur, un réseau de bandes étroites (24) à allongement court, ces bandes étroites étant espacées et réparties en spirale en s'étendant vers le bas depuis le coté intérieur de la chaussette en allant vers son coté extérieur, en contraignant légèrement les muscles jumeaux du mollet,
   - et, entre ces diverses bandes, un textile élastique (25) présentant des mailles à allongement intermédiaire entre long et court.

8. Enveloppe de compression selon la revendication 7 **caractérisée en ce que**, à son extrémité inférieure, chacune des deux bandes larges (23c) s'étend au dessus d'une malléole, latérale ou médiale, et diverge par une bande étroite (23d) longeant le pied en s'étendant au-dessus, respectivement, du tendon fibulaire ou du tendon du muscle tibial antérieur longeant le pied.

9. Enveloppe de compression obtenue par le procédé selon la revendication 1 et l'une quelconque des revendications 2 à 5, cette enveloppe étant réalisée par tricotage avec des fils de trame élastiques procurant une tension circonférentielle et longitudinale et comportant, au moins dans certains segments circulaires juxtaposés, des tronçons de mailles (7a, 7b) dont l'allongement est long et des tronçons de mailles (7a), dont l'allongement est court, ces tronçons (7a, 7b) étant répartis, selon l'action corrective voulue ou selon les besoins d'un traitement, sur les parties de l'enveloppe destinés à venir en regard des diverses zones anatomiques compressibles et déformables (Z2), composées de peau, graisse, muscles (TBV mous), et des zones anatomiques rigides et peu déformables (Z1), composées d'os et tendons (TBV durs), de la partie de corps qu'elle doit entourer, par exemple jambe, cuisse, bras et avant bras, cette répartition des tronçons (7a et 7b) étant réalisée, selon les données du référentiel, pour contrôler la déformation des tissus biologiques contraints et le comportement physique et physiologique des TBV mous par rapport aux TBV durs sous l'effet des compressions transmises par les tronçons de mailles (7a et 7b) à allongement longs et à allongement court, **caractérisée et en ce que**, dans son application à un cuissard d'effort dynamique, l'enveloppe comprend, sur un textile de base (30) ayant un allongement long et s'étendant entre deux zones d'ancrage constituées par une ceinture (31) et des bandeaux de maintien (33), venant sous les genoux :

   - sur sa partie arrière des bandes (37) à allongement court, s'étendant vers le bas depuis la partie centrale de la ceinture (31) en formant deux bras, passant sur les muscles fessiers et se divisant, sur chaque jambe, en deux branches (37a) s'écartant sur les cotés de la jambe jusqu'aux zones d'insertion (38) du semi tendineux et du biceps fémoral,

- sur la partie avant de chaque jambe, d'une part, une bande (39) allant en descendant en biais de la partie latérale extérieure de la ceinture (31) vers la partie intérieure du bandeau (33), d'autre part, une bande latérale interne (40), allant en biais de la ceinture au bord latéral interne du bandeau, et, de plus, trois barreaux horizontaux (42) s'étendant entre les bandes (39 et 40), ces bandes et barreaux ayant un allongement court, qui est plus long que l'allongement court des bandes (37, 37a) passant sur les muscles fessiers.

10. Ensemble d'enveloppes de compression avec contrôle de la déformation des tissus biologiques contraints, chaque enveloppe étant réalisée par tricotage avec des fils de trame élastiques procurant une tension circonférentielle et longitudinale, **caractérisé en ce que** cet ensemble est composé de plusieurs enveloppes présentant des périmètres différents en fonction des morphotypes des sujets, chaque enveloppe présentant, en section transversale, au moins un premier tronçon de mailles (22, 30,) dont l'allongement est long, ce premier tronçon étant destiné à venir au contact des TBV durs, et au moins un second tronçon de mailles (37, 39, 40, 42) dont l'allongement est court, ce second tronçon étant destiné à venir au contact des TBV mous, tandis que, sur au moins une partie de la dimension longitudinale des enveloppes, l'allongement de chaque tronçon court diminue d'une enveloppe à l'autre, lorsque le périmètre de l'enveloppe augmente selon les relations suivantes :

- relation 1 : AC1(M) > AC1(N) > AC1(G) > AC1(O),
- relation 2 : AC2(M) > AC2(N) > AC2(G) > AC2(O),
- relation 1' : AC'1(M) > AC'1(N) > AC'1(G) > AC'1(O), et
- relation 2' : AC'2(M) > AC'2(N) > AC'2(G) > AC'2(O),

relations dans lesquels les notations (M), (N), (G) et (O) indiquent les morphotypes, respectivement, maigre, normal, gras et obèse.

11. Ensemble d'enveloppes de compression selon la revendication 10, **caractérisé en ce que,** sur au moins une partie de la dimension longitudinale des enveloppes, l'allongement de chaque tronçon à allongement long (22, 30) diminue également d'une enveloppe à l'autre, lorsque le périmètre de l'enveloppe augmente.

12. Ensemble d'enveloppes de compression selon la revendication 10 ou 11, **caractérisé en ce qu'**il est constitué de quatre enveloppes de périmètres différents.

13. Utilisation d'un ensemble d'enveloppes de compression selon la revendication précédente, pour des sujets de morphotypes respectivement, maigre (M), normal (N), gras (G) et obèse (O).

**Patentansprüche**

1. Verfahren zur Definition eines elastischen Strumpfes zur Kompression-Fixation zur Änderung lebendes biologischen Gewebes, das eines menschlichen Körperteils, **dadurch gekennzeichnet, dass** es umfasst:

A) einen Schritt:

- der Bestimmung eines Referenzsystems, bestehend aus Tabellen, die für mehrere menschliche Morphotypen, zum Beispiel mager (M), normal (N), dick (G) und fettleibig (O) für verschiedene Teile des menschlichen Körpers, zum Beispiel Bein, Wade, Schenkel, Arm, Vorderarm, Becken und für verschiedene Typen biologischer Gewebe, weich oder hart, die Mindest- und Höchstwerte der Drücke angibt, die auf diesen Teil des menschlichen Körpers durch einen textilen Kompressionsstrumpf (7) ausgeübt werden können, der einen Druck auf den Umfang und in Längsrichtung ausübt, und
- der topographischen Lage der anatomischen Zonen, die weichen und harten lebendem, biologischen Gewebe des zu komprimierenden menschlichen Körpers entsprechen, wie zum Beispiel das Bein, der Schenkel, der Arm, der Vorderarm, das Knie, der Ellbogen, ..., entsprechend der gewünschten Korrekturaktion oder den Behandlungsbedürfnissen,

B) einen Schritt der Anpassung des Strumpfes an die Korrekturbedürfnisse, der darin besteht:

- einerseits die Zonen des textilen Strumpfes mit Zonen mit langer Dehnung (7a) oder mit kurzer Verlängerung (7b) zu definieren, die auf die weichen und harten lebenden, biologischen Gewebe des komprimierten Körpers anzuwenden sind, entsprechend der gewünschten Korrekturaktion oder den Behandlungsbedürfnissen, diese lebenden, biologischen Gewebe definieren die Kenndaten der absorbierenden weichen und verformbaren (Z2) und der harten und wenig verformbaren (Z1) Masse im untersuchten komprimierten Körper,

- andererseits den Morphotyp des Patienten zu definieren, durch anthropometrische Messungen, Messung des Body-Mass-Indexes (IMC) und/ oder Messung der Hautfalte, die letztgenannten Messungen definieren die Eigenschaften (Dichte, Dicke, Verteilung, Konfiguration, anatomische Lokalisierung, Topographie) der untersuchten, komprimierten absorbierenden weichen oder harten Masse im Körper und

- zudem zur Definition der wirksamen Drücke, die zwischen Mindestdruck, Höchstdruck und Verformungsdruck liegen, die auf die weichen und harten Massen des lebenden, biologischen Gewebes anzuwenden sind, in Abhängigkeit von der gewünschten Korrekturaktion oder den Behandlungsbedürfnissen;

C) einen Schritt der Bestimmung der Eigenschaften für die Herstellung des Strumpfes (7), die darin besteht, ausgehend i) von der in dem dem Morphotyp des Patienten entsprechenden Referenzdokument, ii) den persönlichen Eigenschaften des Patienten und iii) der gewünschten Korrekturaktion:

- die Spannung zu bestimmen, die die Wirkfäden erhalten müssen, um im Strumpf (7) die gewünschte Umfangs- und Längsspannung zu erreichen,

- für jede Querschnittsfläche des Strumpfes (7) und über ihre Länge, je nach Anwendung die Zonen mit langer Dehnung (7a) zu verteilen, die auf den widerstandsfähigen und wenig verformbaren Teilen (Z1) (harte lebende, biologische Gewebe) des menschlichen Körperteils aufliegen und die mit kurzer Dehnung (7b) die über den Zonen (Z2) aus weichem, leichter verformbaren Stoff der weichen lebenden, biologischen Gewebe anzuordnen sind, mit dem Zweck der Optimierung des Verhaltens des lebenden, biologischen Gewebes durch Kontrolle der Übertragung von Drücken und Verformungen auf die Gewebe.

2. Verfahren zur Definition einer elastischen Strumpfes zur Kompression-Fixation zur Änderung des Verhaltens von darunter befindlichem, lebendem biologischem Gewebe eines Körperteils, gemäß Anspruch 1 **dadurch gekennzeichnet, dass** es darin besteht, unter vier Morphotypen auszuwählen, also mager, normal, dick und fettleibig.

3. Verfahren zur Definition eines elastischen Strumpfes zur Kompression-Fixation zur Änderung des Verhaltens von darunter befindlichem, lebendem biologischem Gewebe eines Körperteils, gemäß Anspruch 1 **dadurch gekennzeichnet, dass** die Verteilung der Zonen im Strumpf (7), also jeweils Zonen mit langer Dehnung (7a) und Zonen mit kurzer Dehnung (7b) sich aus Änderungen des Deckungsfaktors der Maschen des Gewirks, das den Strumpf (7) bildet, erhalten wird.

4. Verfahren zur Definition eines elastischen Strumpfes zur Kompression-Fixation zur Änderung des Verhaltens von darunter befindlichem, lebendem biologischem Gewebe eines Körperteils, gemäß Anspruch 1 **dadurch gekennzeichnet, dass** die Verteilung der Zonen in der Strumpf (7), also jeweils Zonen mit langer Dehnung (7a) und Zonen mit kurzer Dehnung (7b) sich aus Änderungen der Eigenschaften des Schussfadens ergibt.

5. Verfahren zur Definition eines elastischen Strumpfes zur Kompression-Fixation zur Änderung des darunter befindlichem, lebenden biologischen Gewebes eines menschlichen Körperteils, gemäß Anspruch 1 **dadurch gekennzeichnet, dass** die Verteilung der Zonen im Strumpf (7) Zonen mit langer Dehnung (7a) und Zonen mit kurzer Dehnung (7b) erhalten wird durch Hinzufügen, mit dem Stickverfahren, von zusätzlichen Maschen auf einem Oberflächensegment.

6. Verfahren zur Definition eines elastischen Strumpfes zur Kompression-Fixation zur Änderung des darunter befindlichem, lebenden biologischen Gewebes eines menschlichen Körperteils, gemäß Anspruch 1 **dadurch gekennzeichnet, dass** ein Hauptmorphotyp des Patienten definiert wird, sowie ein lokaler Morphotyp dieses Patienten, der sich vom Hauptmorphotyp unterscheidet, auf Ebene eines Traumabereichs und dass der Strumpf spezifische Dehnungswerte im Bereich dieses Strumpfes enthält, die auf diesem Traumabereich des Patienten aufliegen.

7. Elastischer Kompressionsstrumpf, erhalten nach den Verfahren gemäß Anspruch 1 und irgendeinem anderen der Ansprüche 2 bis 5, dieser Strumpf wird hergestellt durch Stricken mit elastischen Schussfäden, die für eine Spannung ins Umfangs- und in Längsrichtung sorgen, und die, zumindest in bestimmten hintereinanderliegenden Ringsegmenten Maschenteilabschnitte (7a, 7b) enthalten, deren Dehnung lang ist und Maschenteilabschnitte (7a) deren Dehnung kurz ist, diese Teilabschnitte (7a, 7b) sind entsprechend der gewünschten Korrekturaktion oder den Behandlungsbedürfnissen über die Teile des Strumpfes verteilt, die auf verschiedenen, anatomisch komprimierbaren und verformbaren (Z2) Zonen aufliegen, die aus Haut, Fett und Muskeln (Weichgewebe) bestehen und aus anatomisch starren und wenig verformbaren (Z1) anatomischen Zonen, bestehend aus Knochen und Sehnen (Hartgewebe) des Körperteils, den der Strumpf umgeben soll, zum Beispiel Bein, Schenkel, Arm und Vorderarm, diese Verteilung der Teilabschnitte (7a und 7b) erfolgt nach den Daten des Referenzdokumentes, zur Kontrolle der Verformung der zusammengepressten biologischen Geweben und dem physischen und physiologischen Verhalten der Weichgewebe gegenüber den Hartgeweben unter der Wirkung der Kompression durch die Maschenteilabschnitte (7a und 7b) mit kurzer oder langer Dehnung, **gekennzeichnet dadurch, dass** bei der Anwendung für einen Wadenstrumpf zur Behandlung der arteriellen Verschlusskrankheit der unteren Gliedmaßen der Strumpf umfasst:

- einen Textilstreifen mit langer Dehnung (22), dazu bestimmt auf der Vorderseite des Beines und dem Hartgewebe aufzuliegen,
- zwei Streifen (23a) mit kurzer Dehnung neben dem (22) mit langer Dehnung, die sich durch breite Streifen (23b) verlängern, die einerseits und auf der Rückseite in For eines oberen X, die Wade umrahmen und auf der Außenseite stärkeren Drück ausüben und andererseits im unteren Bereich und über Verzweigungen (23c), ein weiteres X bilden, das die Verbindung des Fersenbeins und der Spitze des Soleux bedeckt.
- in dem Intervall zwischen den Verzweigungen (23b), die das obere X bilden, ein Netz aus schmalen Streifen (24) mit kurzer Dehnung, diese schmalen Streifen haben einen Abstand und sind spiralförmig verteilt, wobei sie nach unten verlaufen, von der Innenseite der Wadenstrumpfs aus zur Außenseite, wobei die Zwillingsmuskeln der Wade leicht zusammengepresst werden,
- und zwischen diesen verschiedenen Streifen, ein dehnbares Textil (25) mit Maschen mit mittlerer Dehnung zwischen lang und kurz.

8. Kompressionsstrumpf gemäß Anspruch 7, **dadurch gekennzeichnet, dass** in seinem unteren Bereich jeder der beiden breiten Streifen (23c) sich über einen lateralen oder medialen Knöchel erstreckt und durch einen schmalen Streifen (23d) divergiert, der entlang des Fußes verläuft und jeweils über der fibularen Sehen oder der Sehne des vorderen Schienbeinmuskels entlang des Fußes liegt.

9. Elastischer Kompressionstrumpf, erhalten nach den Verfahren gemäß Anspruch 1 und irgendeinem anderen der Ansprüche 2 bis 5, dieser Strumpf wird hergestellt durch Stricken mit elastischen Schussfäden, die für eine Spannung in Umfangs- und in Längsrichtung sorgen, und die, zumindest in bestimmten hintereinanderliegenden Ringsegmenten Maschenteilabschnitte (7a, 7b) enthalten, deren Dehnung lang ist und Maschenteilabschnitte (7a) deren Dehnung kurz ist, diese Teilabschnitte (7a, 7b) sind entsprechend der gewünschten Korrekturaktion oder den Behandlungsbedürfnissen über die Teile des Strumpfes verteilt, die auf verschiedenen, anatomisch komprimierbaren und verformbaren (Z2) Zonen aufliegen, die aus Haut, Fett und Muskeln (Weichgewebe) bestehen und aus anatomisch starren und wenig verformbaren (Z1) anatomischen Zonen, bestehend aus Knochen und Sehnen (Hartgewebe) des Körperteils, den die Strumpf umgeben soll, zum Beispiel Bein, Schenkel, Arm und Vorderarm, diese Verteilung der Teilabschnitte (7a und 7b) erfolgt nach den Daten des Referenzdokumentes, zur Kontrolle der Verformung der zusammengepressten biologischen Geweben und dem physischen und physiologischen Verhalten der Weichgewebe gegenüber den Hartgeweben unter der Wirkung der Kompression durch die Maschenteilabschnitte (7a und 7b) mit kurzer oder langer Dehnung, und **dadurch gekennzeichnet, dass** bei der Anwendung auf einen Gurt mit dynamischer Beanspruchung, der Strumpf, auf einer textilen Basis (30) mit langer Dehnung, die sich zwischen zwei Verankerungszonen befindet, die einen Gürtel (31) und Haltestreifen (33) umfassen, die unter die Knie reichen:

- im hinteren Teil Streifen (37) mit kurzer Dehnung umfasst, nach unten verlaufend von dem Mittelteil des Gürtels (31) aus und dabei zwei Arme bildend, die über die Gesäßmuskel verlaufen und die sich über jedem Bein in zwei Verzweigungen (37a) teilen, die sich über die Seiten des Beins bis zu den Ansatzzonen (38) des Semitendinosus und des Biceps Femoris erstrecken.
- und auf der Vorderseite jedes Beins, einerseits einen Streifen (39), der schräg über die laterale Außenseite des Gürtel (31) hinabführt zur Innenseite des Streifens (33), andererseits eine laterale Innenseite (40), die schräg vom Gürtel zur lateralen Innenseite des Streifens führen und außerdem drei waagrechte Stäbe (42), die zwischen den Streifen (39 und 40) verlaufen, diese Streifen und Stäbe haben eine kurze Dehnung, die länger ist als die kurze Dehnung der Streifen (37, 37a) und die über die Gesäßmuskeln verlaufen.

10. Einheit aus Kompressionsstrümpfen mit Kontrolle der Verformung der komprimierten biologischen Gewebe, jeder Strumpf wird hergestellt durch Stricken mit elastischen Schussfäden, die für eine Spannung ins Umfangs- und in Längsrichtung sorgen, **dadurch gekennzeichnet, dass** diese Einheit aus mehreren Strümpfen besteht, die verschiedene Perimeter aufweisen, in Abhängigkeit von den Morphotypen der Patienten, jeder Strumpf weist, in der Querschnittsfläche, mindestens einen ersten Maschenteilabschnitt (22, 30) auf, dessen Dehnung lang ist, wobei dieser Abschnitt in Kontakt mit den Hartgeweben kommen soll und mindestens einen zweiten Maschenteilabschnitt (37, 39, 40, 42) mit kurzer Dehnung, wobei dieser zweite Abschnitt in Kontakt mit den Weichgeweben kommen soll, während, auf mindestens einem Teil der Längsabmessung der Strümpfe, sich die kurze Dehnung jedes Abschnitts von einem Strumpf zum nächsten verringert, wenn der Perimeter des Strumpfes ansteigt, im folgenden Verhältnis:

- Verhältnis 1: AC1(M) > AC1(N) > AC1(G) > AC1(O),
- Verhältnis 2: AC2(M) > AC2(N) > AC2(G) > AC2(O),
- Verhältnis 1': AC'1(M) > AC'1(N) > AC'1(G) > AC'1(O), und
- Verhältnis 2': AC'2(M) > AC'2(N) > AC'2(G) > AC'2(O),

In diesen Verhältnissen weisen die Bezeichnungen (M), (N), (G) und (O) auf die Morphotypen, also mager, normal, dick und fettleibig hin.

11. Einheit aus Kompressionsstrümpfen, gemäß Anspruch 10, **dadurch gekennzeichnet, dass** auf mindestens einem Teil der Längsabmessung der Strümpfe, sich die Dehnung jedes Abschnitts mit langer Dehnung (22, 30) ebenfalls von einem Strumpf zum nächsten verringert, wenn der Perimeter des Strumpfes ansteigt.

12. Einheit aus Kompressionsstrümpfen, gemäß Anspruch 10 oder 11, **dadurch gekennzeichnet, dass** sie aus vier Strümpfen mit unterschiedlichem Umfang besteht.

13. Einsatz einer Einheit aus Kompressionsstrümpfen, gemäß dem vorstehenden Anspruch für Patienten mit den jeweiligen Morphotypen mager (M), normal (N), dick (G) und fettleibig (O).

**Claims**

1. Process for producing an elastic compressing/containing jacket modifying the behavior of living biological tissues (LBTs) underlying part of the human body, **characterized in that** it includes:

A) one phase:

- for establishing a baseline composed of tables stating, for multiple human morphology types, such as thin (M), normal (N), fat (G) and obese (O), for several parts of the human body, such as the leg, calf, thigh, arm, forearm and pelvis, and for multiple types of biological tissue, whether soft or hard, the minimum and maximum values of the pressures that can be applied to this part of the human body by a textile compression jacket (7) exerting a circumferential and longitudinal pressure; and
- topographic localization of the corresponding anatomic areas of these soft and hard LBTs of the part of the human body to be compressed, such as - for example
- the leg, the thigh, the arm, the forearm, the knee, the elbow, etc., according to the desired corrective action or the needs of treatments;

B) one phase of adaptation of the jacket to the corrective needs, consisting of:

- firstly, defining the areas of the textile jacket with long extension (7a) or short extension (7b) to be applied to the soft or hard LBTs of the compressed body, according to the desired corrective action or the needs of treatments, with these LBTs defining the characteristics of the soft and deformable absorbent mass (Z2) and hard and little-deformable absorbent mass (Z1) in the compressed body under examination;
- secondly, establishing the morphological type of the subject by means of anthropometrical measurements, measurement of the body mass index (BMI), and/or measurement of the skinfold, with these latter measurements defining the characteristics (density, thickness, distribution, configuration, anatomical location, topography) of the soft or hard absorbent mass in the compressed body under examination; and
- additionally, defining the value of the effective pressures included between the minimum, maximum and deformation pressures to be applied to the soft or hard masses of LBTs, according to the desired corrective action or the needs of treatments;

C) one phase of establishment of the characteristics for production of the jacket (7) consisting in, using i) data taken from the corresponding baseline for the subject's morphological type, ii) the subject's own data and iii) data specific to the desired corrective action:

- determining the tension to be applied on the weave yarns to endow the jacket (7) with the desired circumferential and longitudinal tension; and
- for each transversal section of the jacket (7), and over its length, distributing - according to the application - the areas of the jacket with long extension (7a) above the resistant and little-deformable parts (Z1) of that part of the human body (hard LBTs), and those with short extension (7b) above the areas (Z2) of the soft and more-easily-deformable matters of the soft LBTs, to optimize the behavior of the LBTs by controlling the transmission of pressures and deformations within the LBTs.

2. Process for producing an elastic compressing/containing jacket modifying the behavior of the underlying biological tissues (LBTs) of a part of the human body, in accordance with claim 1, **characterized in that** it consists in choosing between four morphological types, being respectively thin, normal, fat and obese.

3. Process for producing an elastic compressing/containing jacket modifying the behavior of the underlying biological tissues (LBTs) of a part of the human body, in accordance with claim 1, **characterized in that** the distribution, within the jacket (7) of the respective areas with long extension (7a) and areas with short extension (7b) is obtained through modifications of the factor of coverage of the meshes of the weave constituting the jacket (7).

4. Process for producing an elastic compressing/containing jacket modifying the behavior of the underlying biological tissues (LBTs) of a part of the human body, in accordance with claim 1, **characterized in that** the distribution, within the jacket (7) of the respective areas with long extension (7a) and areas with short extension (7b) is obtained through modifications in the characteristics of the weft yarn.

5. Process for producing an elastic compressing/containing jacket modifying the behavior of the underlying biological tissues (LBTs) of a part of the human body, in accordance with claim 1, **characterized in that** the distribution within the jacket (7) of the respective areas with long extension (7a) and areas with short extension (7b) is obtained by addition, through the embroidery technique, of additional meshes over a segment of the surface.

6. Process for producing an elastic compressing/containing jacket modifying the behavior of the underlying biological tissues (LBTs) of a part of the human body, in accordance with claim 1, **characterized in that** one defines a principal morphological type of the subject, together with a local morphological type of this subject, being different from the principal morphological type, at a traumatic area, and one gives the jacket specific extension values within the region of this jacket, intended to cover the subject's traumatic area.

7. Compression jacket produced through the process in accordance with claim 1 and with any one of claims 2 to 5, with this jacket being produced via weaving with elastic weft yarns endowing a circumferential tension and a longitudinal tension, and incorporating - in at least certain juxtaposed circular segments - sections of meshes (7a, 7b) of which the extension is long and sections of meshes (7a) of which the extension is short, with these sections (7a, 7b) being distributed according to the desired corrective action or according to the needs of a treatment, over the parts of the jacket intended to be positioned opposite various compressible and deformable (Z2) anatomic areas composed of skin, fat, muscles (soft LBTs), and rigid and little-deformable (Z1) anatomic areas, composed of bone and tendons (hard LBTs) of the part of the body that it is supposed to surround, such as the leg, thigh, arm and forearm, with this distribution of the sections (7a and 7b) being produced in accordance with the data in the baseline, to control the deformation of the constrained biological tissues and the physical and physiological behavior of the soft LBTs in relation to the hard LBTs, under the effect of the compressions transmitted by the sections of meshes (7a and 7b) with long extension and with short extension, **characterized in that,** in its application to a sock for influencing the arteriopathy of the lower members, the jacket incorporates:

- one textile strap with long extension (22) intended to press against the front of the leg and the hard LBTs;
- two straps (23a) with short extension next to the strap (22) with long extension, and extended by wide straps (23b) forming - firstly, and on the rear part - an upper X enclosing the calf and constraining it more strongly on its exterior side and - secondly, and on the lower part, with branches (23c) - another X covering the join between the calcaneus and the tip of the soleus;
- in the gap between the branches (23b) forming an upper X, a network of narrow straps (24) with short extension, with these narrow straps being spaced and distributed in spiral form, extending downwards from the interior side of the sock towards its exterior side, and lightly constraining the gemellus muscles of the calf;
- and, between these various straps, an elastic textile (25) having meshes with intermediate extension between long and short.

8. Compression jacket in accordance with claim 7, **characterized in that,** at its lower extremity, each of the two wide straps (23c) extend above a lateral or median malleolus and diverge via a narrow strap (23d) extending along the foot while extending above, respectively, the fibular tendon or the tendon of the anterior tibial muscle along the foot.

9. Compression jacket produced via the process according to claim 1 and any one of claims 2 to 5, with this jacket being produced by weaving with elastic weft yarns endowing a circumferential and longitudinal tension, and incorporating - at least in certain juxtaposed circular segments - sections of meshes (7a, 7b) of which the extension is long, and sections of meshes (7a) of which the extension is short, with these sections (7a, 7b) being distributed according to the desired corrective action or according to the needs of a treatment, over the parts of the jacket intended to be positioned opposite various compressible and deformable anatomic areas (Z2), composed of skin, fat, muscles (soft LBTs), and rigid and little-deformable anatomic areas (Z1), composed of bone and tendons (hard LBTs), of the part of the body that it is supposed to surround - for example, the leg, thigh, arm and forearm - with this distribution of the sections of meshes (7a and 7b) being produced in accordance with the data from the baseline, to control the deformation of the biological tissues constrained and the physical and physiological behavior of the soft LBTs in relation to the hard LBTs under the effect of the compressions transmitted by the sections of meshes (7a and 7b) with long extension and short extension, **characterized in that,** in its application to a dynamic-stress thigh band, the jacket includes, on a textile base (30) having a long extension and extending between two areas of anchoring constituted by a belt (31) and securing straps (33) coming under the knees:

   - on its rear part, straps (37) with short extension, extending towards the bottom from the central part of the belt (31), forming two arms, passing over the gluteus muscles and dividing, on each leg, into two branches (37a) splitting on the sides of the leg, until the insertion areas (38) of the semitendinosus and the biceps femoris;
   - on the front part of each leg, firstly, one strap (39) descending obliquely from the exterior lateral part of the belt (31) to the interior part of the strap (33) plus, secondly, an internal lateral strap (40) descending obliquely from the belt on the internal lateral edge of the band and, in addition, three horizontal bars (42) extending between the straps (30 and 40), with these straps and bars having a short extension that is longer than the short extension of the straps (37, 37a) passing over the gluteus muscles.

10. Set of compression jackets with control over the deformation of the constrained biological tissues, with each jacket being produced via weaving with elastic weft yarns endowing a circumferential and longitudinal tension, **characterized in that** this ensemble composed of multiple jackets having different perimeters according to the morphological types of the subjects, with each jacket having - in its transversal section - at least one first section of meshes (22; 30) of which the extension is long, with this first section being intended to come into contact with the hard LBTs, and at least one second section of meshes (37; 39; 40; 42) of which the extension is short, with this second section being intended to come into contact with the soft LBTs, while - over at least one part of the longitudinal dimension of the jackets - the extension of each short section decreases from one jacket to another, when the perimeter of the jacket increases in accordance with the following relationships:

   - relationship 1: AC1(M) > AC1(N) > (AC1(G) > AC1(O);
   - relationship 2: AC2(M) > AC2(N) > (AC2(G) > AC2(O);
   - relationship 1: AC1(M) > AC1(N) > (AC1(G) > AC1(O); and
   - relationship 2: AC2(M) > AC2(N) > (AC2(G) > AC2(O);

   in which relationships the notations (M), (N), (G) and (O) stand for the morphological types, being respectively thin, normal, fat and obese.

11. Set of compression jackets in accordance with claim 10, **characterized in that,** on at least one part of the longitudinal dimension of the jackets, the extension of each section with short extension (22; 30) also decreases from one jacket to another, when the perimeter of the jacket increases.

12. Set of compression jackets in accordance with claim 10 or claim 11, **characterized in that** it is composed of four jackets with different perimeters.

13. Use of a set of compression jackets in accordance with the preceding claim, for subjects with morphological types being respectively thin (M), normal (N), fat (G) and obese (O).

**FIG. 1**

FIG. 2

FIG.5

FIG.8

FIG. 3

FIG. 6

FIG. 9

FIG.4

FIG.7

FIG.10

## FIG. 11

## FIG. 12

## FIG. 13

## FIG. 14

FIG.15

FIG. 16

FIG.17

**FIG. 18**

**FIG.19**

FIG. 20

FIG. 21

FIG. 22

FIG. 23

**FIG.24**

**FIG.25**

**FIG. 26**

## FIG. 27

| MOLLET | | |
|---|---|---|
| SUJET | Pressions Minimales en mm de Hg | Pressions Maximales en mm de Hg |
| MAIGRE | 5 à 7 | 90 |
| NORMAL | 5 à 8 | 110 |
| GRAS | 5 à 10 | 120 |
| OBESE 1 | 5 à 12 | 140 |
| OBESE 2 | 5 à15 | 160 |

## FIG. 28

| BRAS | | |
|---|---|---|
| SUJET | Pressions Minimales en mm de Hg | Pressions Maximales en mm de Hg |
| MAIGRE | 5 à 7 | 80 |
| NORMAL | 5 à 8 | 90 |
| GRAS | 5 à 10 | 110 |
| OBESE 1 | 5 à 12 | 130 |
| OBESE 2 | 5 à15 | 150 |

**FIG. 29**

22
23b
XXXIII
24
23b
23a
22a

23b
25
25
23b
23a
25
22b

**FIG. 30**

25
24
23b
23c
22b
23d
23a
25
22
22a

**FIG. 31**

23b
24
23b
25
23c
25
24
23c
22b

**FIG. 32**

25
23a
22
25
22a
22b
23b
25
24
25
23b
23c
23d
22b

FIG. 33

FIG. 34

FIG. 39

FIG. 40

FIG. 35

FIG. 36

FIG. 37

FIG. 38

**EP 3 010 461 B1**

## RÉFÉRENCES CITÉES DANS LA DESCRIPTION

### Documents brevets cités dans la description

- WO 2005106087 A
- EP 1194071 A